# EUROPEAN PATENT APPLICATION

(11) **EP 4 737 439 A1**
(43) Date of publication of application: **06.05.2026**
(21) Application number: 24830546.8
(22) Date of filing: 17.06.2024
(51) Int. Cl.: C07D 207/14, A61K 9/51, A61K 47/22, A61P 35/00

(54) **IONIZABLE LIPID AND USE THEREOF**

(30) Priority: 29.06.2023 CN 202310778315
(71) Applicant: Axter Therapeutics (Beijing) Co., Ltd., Daxing District, Beijing 100023 (CN)
(72) Inventor: ZHANG, Yuanyuan, Beijing 100023 (CN); LI, Ke, Beijing 100023 (CN); GU, Hanqing, Beijing 100023 (CN); ZHANG, Huicong, Beijing 100023 (CN); SONG, Jin, Beijing 100023 (CN); HUANG, Huiya, Beijing 100023 (CN); XU, Zengjun, Beijing 100023 (CN)
(74) Representative: Elzaburu S.L.P.
(86) International application number: PCT/CN2024/099640
(87) International publication number: WO 2025/001900

(57) **Abstract**

The present invention provides an ionizable lipid and a drug delivery system comprising the ionizable lipid. Specifically, the present invention provides an ionizable lipid having the structure of formula (1), or a pharmaceutically acceptable salt, tautomer or stereoisomer thereof. Lipid nanoparticles constructed by using the ionizable lipid can realize safe and efficient delivery of nucleic acid drugs, small molecule drugs, peptide drugs and protein drugs.

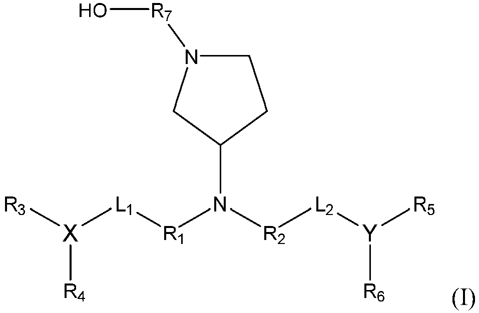

## Description

### Technical Field

The present disclosure relates to the field of biomedicine, and specifically relates to an ionizable lipid and use thereof in drug delivery.

### Background

In recent years, messenger RNA drugs have become a key therapeutic means for preventing and treating infectious diseases and tumors. The messenger RNA drug technology has been recognized by the industry. Benefiting from its short research and development cycle, low risk of insertional mutagenesis and diversity in encoding proteins. mRNA is highly suitable for the development of vaccines or therapeutic drugs. However, mRNA itself is highly unstable and susceptible to degradation by ubiquitous RNases. Additionally, due to the inherent negative charge and large molecular weight (typically greater than 10⁶ Da) of mRNA, mRNA molecules are restricted from entering cells. Therefore, the development of appropriate delivery carriers to protect fragile mRNA molecules and deliver them into the cytoplasm holds great significance.

Currently, a variety of mRNA delivery carriers have been developed, including lipid nanoparticles (LNPs), inorganic nanoparticles, polymeric nanoparticles, viral vectors and exosomes, etc. At present, LNPs are widely used as drug delivery carriers, and their main components include ionizable lipids, phospholipids, cholesterol and polyethylene glycol-containing lipids. The most important component in LNPs is the ionizable lipid. Early permanently positively charged cationic lipids exhibit short *in vivo* circulation time, high toxicity and severe allergic reactions, which is because their inherent positive charge causes them to adsorb proteins during *in vivo* circulation, making them liable to be captured and cleared by the reticuloendothelial system. The inherent positive charge interacts with negatively charged cell membranes, causing membrane destabilization and subsequent severe toxicity; permanently positively charged cationic lipids can activate the complement system, leading to allergic reactions. Ionizable lipids are electrically neutral under physiological pH conditions. Therefore, LNPs prepared from ionizable lipids exhibit relatively high safety. The ionizable lipid endows the LNP with lysosomal escape capability, and through the proton sponge effect and membrane fusion mechanism, enables the LNP to escape and release mRNA into the cytoplasm, where the mRNA binds to the protein-encoding ribosomes for translation of the encoded protein.

In short, the development of a suitable ionizable lipid is one of the keys for developing LNPs with high safety and high lysosomal escape efficiency.

Therefore, it is of great significance to develop an ionizable lipid with low toxicity and high delivery efficiency.

### Summary

The present disclosure provides an ionizable lipid with low toxicity and high delivery efficiency.

In a first aspect of the present disclosure, provides an ionizable lipid, or a pharmaceutically acceptable salt, a tautomer or a stereoisomer thereof, wherein, the ionizable lipid has the structure of Formula I below: 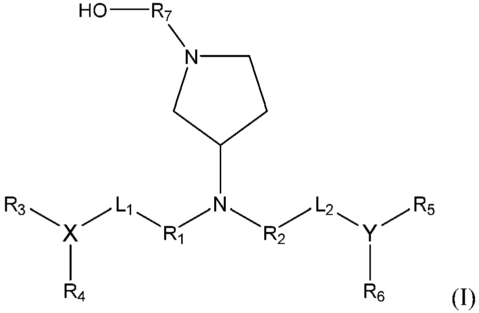 wherein,
R₁ and R₂ are each independently selected from -(CH₂)ₙ-, wherein n is a positive integer ranging from 1 to 14;
X and Y are each independently -CH- or N;
L₁ has the structure of -(L₁ₐ-L_{1b})- from right to left, or is absent, wherein, L₁ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, preferably selected from -O-, -(C=O)O-, -O(C=O)-, -CH(OH)-; L_{1b} is -(CH₂)ₙ-, wherein n is selected from 0, 1, 2, 3 or 4;
L₂ has the structure of -(L₂ₐ-L_{2b})- from left to right, or is absent; wherein, L₂ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, preferably selected from -O-, -(C=O)O-, -O(C=O)-, -CH(OH)-; L_{2b} is -(CH₂)ₙ-, wherein n is selected from 0, 1, 2, 3 or 4;
R₃, R₄, R₅ and R₆ are each independently H, CH₃, C₂-C₃₀ hydrocarbyl group (e.g., C₂-C₃₀ alkyl, C₂-C₃₀ alkenyl, C₂-C₃₀ alkynyl), or -(CH₂)ₛ-R^{a}-(CH₂)_{g}-R^{b}-(CH₂)ₘ-CH₃, wherein, s, g are each independently selected from a positive integer ranging from 1 to 20, m is selected from a integer ranging from 0 to 20, preferably s+g+m is 2-35;
R^{a}, R^{b} are each independently absent or selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, preferably selected from -O-, -(C=O)O-, -O(C=O)-, -CH(OH)-;
and, R₃ and R₄ are not both H; and R₅ and R₆ are not both H;
R₇ is selected from C₁-C₅ hydrocarbyl group, or -(CH₂)ₘO(CH₂)ₙ-, wherein m, n are each independently selected from 1, 2, 3.

In another preferred embodiment, the R₃, R₄, R₅ and R₆ are each independently C₄-C₃₀ hydrocarbyl group (e.g., C₄-C₃₀ alkyl, C₄-C₃₀ alkenyl, C₄-C₃₀ alkynyl), preferably is C₄-C₂₀ hydrocarbyl group (e.g., C₄-C₂₀ alkyl, C₄-C₂₀ alkenyl, C₄-C₂₀ alkynyl).

In another preferred embodiment, at least 2, 3, or 4 of the R₃, R₄, R₅ and R₆ are C2-C30 hydrocarbyl group (e.g., C₂-C₃₀ alkyl, C₂-C₃₀ alkenyl, C₂-C₃₀ alkynyl), or -(CH₂)ₛ-R^{a}-(CH₂)_{g}-R^{b}-(CH₂)ₘ-CH₃, wherein s, g, m, R^{a} and R^{b} are defined as above.

In another preferred embodiment, the s+g+m is 3-20, more preferably is 4-15.

In another preferred embodiment, the R₃ has the structure of -R₃ₐ-R_{3b}-R_{3c}-R_{3d}-R₃ₑ,
wherein, R₃ₐ and R_{3c} are each independently -(CH₂)ₙ-, wherein n is selected from a positive integer ranging from 1 to 14;
R_{3b} and R_{3d} are each independently absent or selected from the following functional groups: -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, -(C=C)-(CH₂)-(C=C)-, -(C=C)-, -CH₂-, -(C=C)-, preferably -(C=O)O-, -O(C=O)-, -(S-S)-, -(C=C)-(CH₂)-(C=C)-, -CH(OH)-;
R₃ₑ is C₂-C₂₀ hydrocarbyl group.

In another preferred embodiment, the R₄ has the structure of -R₄ₐ-R_{4b}-R_{4c}-R_{4d}-R₄ₑ;
wherein, R₄ₐ and R_{4c} are each independently -(CH₂)ₙ-, wherein n is selected from a positive integer ranging from 1 to 14;
R_{4b} and R_{4d} are each independently absent or selected from the following functional groups: -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, -(C=C)-(CH₂)-(C=C)-, -(C=C)-, -CH₂-, -(C≡C)-, preferably -(C=O)O-, -O(C=O)-, -(S-S)-, -(C=C)-(CH₂)-(C=C)-, -CH(OH)-;
R_{4c} is C₂-C₂₀ hydrocarbyl group.

In another preferred embodiment, the R₅ has the structure of -R₅ₐ-R_{5b}-R_{5c}-R_{5d}-R₅ₑ,
wherein, R₅ₐ and R_{5c} are each independently -(CH₂)ₙ-, wherein n is selected from a positive integer ranging from 1 to 14;
R_{5b} and R_{5d} are each independently absent or selected from the following functional groups: O, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, -(C=C)-(CH₂)-(C=C)-, -(C=C)-, -CH₂-, -(C=C)-, preferably -(C=O)O-, -O(C=O)-, -(S-S)-, -(C=C)-(CH₂)-(C=C)-, -CH(OH)-;
R₅ₑ is C₂-C₂₀ hydrocarbyl group.

In another preferred embodiment, the R₆ has the structure of -R₆ₐ-R_{6b}-R_{6c}-R_{6d}-R₆ₑ;
wherein, R₆ₐ and R_{6c} are each independently -(CH₂)ₙ-, wherein n is selected from a positive integer ranging from 1 to 14;
R_{6b} and R_{6d} are each independently absent or selected from the following functional groups: O, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, -(C=C)-(CH₂)-(C=C)-, -(C=C)-, -CH₂-, -(C≡C)-, preferably -(C=O)O-, -O(C=O)-, -(S-S)-, -(C=C)-(CH₂)-(C=C)-, -CH(OH)-;
R₆ₑ is C₂-C₂₀ hydrocarbyl group;
R₇ is selected from C₁-C₅ hydrocarbyl group, or -(CH₂)ₘO(CH₂)ₙ-, wherein m, n are each independently selected from 1, 2, 3.

In another preferred embodiment, the X and Y are -CH-.

In another preferred embodiment, the X and Y are N.

In another preferred embodiment, the ionizable lipid has the structure represented by the Formula below:

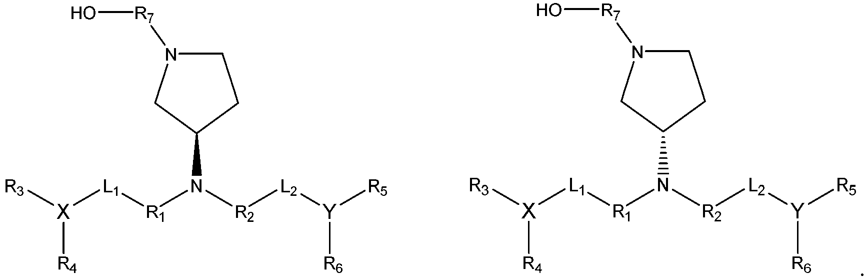

In another preferred embodiment, the ionizable lipid has the substructure represented by Formula (I-1) below: 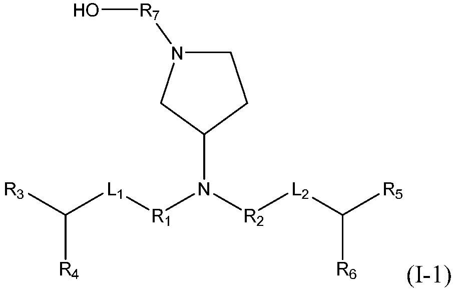 wherein,
R₁ and R₂ are each independently selected from -(CH₂)ₙ-, wherein n is a positive integer ranging from 1 to 14;
L₁ has the structure of -(L₁ₐ-L_{1b})- from right to left, or is absent, wherein, L₁ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, preferably selected from -O-, -(C=O)O-, -O(C=O)-, -CH(OH)-; L_{1b} is -(CH₂)ₙ-, wherein n is selected from 0, 1, 2, 3 or 4;
L₂ has the structure of -(L₂ₐ-L_{2b})- from left to right, or is absent; wherein, L₂ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-; preferably selected from -O-, -(C=O)O-, -O(C=O)-, -CH(OH)-; L_{2b} is -(CH₂)ₙ-, wherein n is selected from 0, 1, 2, 3 or 4;
R₃, R₄, R₅ and R₆ are each independently C₂-C₂₀ hydrocarbyl group;
R₇ is selected from C₁-C₅ hydrocarbyl group, or -(CH₂)ₘO(CH₂)ₙ-, wherein m, n are each independently selected from 1, 2, 3. In another preferred embodiment, the X is N, Y is -CH-.

In another preferred embodiment, the R₆ is H.

In another preferred embodiment, the ionizable lipid has the structure represented by Formula (I-2) below: 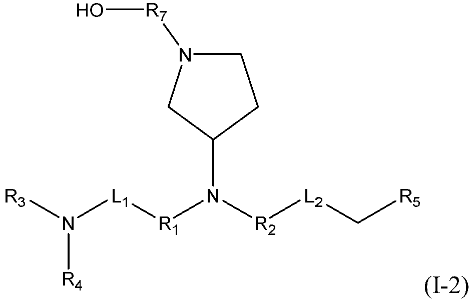 wherein,
R₁ and R₂ are each independently selected from -(CH₂)ₙ-, wherein n is a positive integer ranging from 1 to 14;
L₁ has the structure of -(L₁ₐ-L_{1b})- from right to left, or is absent, wherein, L₁ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, preferably selected from -O-, -(C=O)O-, -O(C=O)-, -CH(OH)-; L_{1b} is -(CH₂)ₙ-, wherein n is selected from 0, 1, 2, 3 or 4;
L₂ has the structure of -(L₂ₐ-L_{2b})- from left to right, or is absent; wherein, L₂ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, preferably selected from -O-, -(C=O)O-, -O(C=O)-, -CH(OH)-; L_{2b} is -(CH₂)ₙ-, wherein n is selected from 0, 1, 2, 3 or 4;
R₃ has the structure of -R₃ₐ-R_{3b}-R_{3c}-R_{3d}-R₃ₑ, R₄ has the structure of -R₄ₐ-R_{4b}-R_{4c}-R_{4d}-R₄ₑ, R₅ has the structure of -R₅ₐ-R_{5b}-R_{5c}-R_{5d}-R₅ₑ;
wherein, R₃ₐ, R_{3c}, R₄ₐ, R_{4c}, R₅ₐ, R_{5c} are each independently -(CH₂)ₙ-, wherein n is selected from a positive integer ranging from 1 to 14;
R_{3b}, R_{3d}, R_{4b}, R_{4d}, R_{5b}, R_{5d} are each independently absent or selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, -(C=C)-(CH₂)-(C=C)-, -(C=C)-, -CH₂-, -(C=C)-, preferably -(C=O)O-, -O(C=O)-, -(S-S)-, -(C=C)-(CH₂)-(C=C)-, -CH(OH)-;
R₃ₑ, R₄ₑ, R₅ₑ are each independently C₂-C₂₀ hydrocarbyl group;
R₇ is selected from C₁-C₅ hydrocarbyl group, or -(CH₂)ₘO(CH₂)ₙ-, wherein m, n are each independently selected from 1, 2, 3.

In another preferred embodiment, the ionizable lipid has the structure represented by Formula (I-3) below: 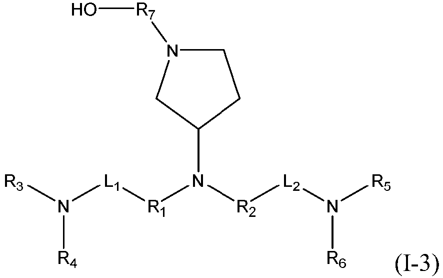 wherein,
R₁ and R₂ are each independently selected from -(CH₂)ₙ-, wherein n is a positive integer ranging from 1 to 14;
L₁ has the structure of -(L₁ₐ-L_{1b})- from right to left, or is absent, wherein, L₁ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, preferably selected from -O-, -(C=O)O-, -O(C=O)-, -CH(OH)-; L_{1b} is -(CH₂)ₙ-, wherein n is selected from 0, 1, 2, 3 or 4;
L₂ has the structure of -(L₂ₐ-L_{2b})- from left to right, or is absent; wherein, L₂ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, preferably selected from -O-, -(C=O)O-, -O(C=O)-, -CH(OH)-; L_{2b} is -(CH₂)ₙ-, wherein n is selected from 0, 1, 2, 3 or 4;
R₃ has the structure of -R₃ₐ-R_{3b}-R_{3c}-R_{3d}-R₃ₑ, R₄ has the structure of -R₄ₐ-R_{4b}-R_{4c}-R_{4d}-R_{4c}; R₅ has the structure of -R₅ₐ-R_{5b}-R_{5c}-R_{5d}-R₅ₑ, R₆ has the structure of -R₆ₐ-R_{6b}-R_{6c}-R_{6d}-R₆ₑ;
wherein, R₃ₐ, R_{3c}, R₄ₐ, R_{4c}, R₅ₐ, R_{5c}, R₆ₐ and R_{6c} are each independently -(CH₂)ₙ-, wherein n is selected from a positive integer ranging from 1 to 14;
R_{3b}, R_{3d}, R_{4b}, R₄ₐ, R_{5b}, R_{5d}, R_{6b} and R_{6d} are each independently absent or selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, -(C=C)-(CH₂)-(C=C)-, -(C=C)-, -CH₂-, -(C≡C)-, preferably -(C=O)O-, -O(C=O)-, -(S-S)-, -(C=C)-(CH₂)-(C=C)-, -CH(OH)-;
R₃ₑ, R₄ₑ, R₅ₑ, R₆ₑ are each independently C₂-C₂₀ hydrocarbyl group;
R₇ is selected from C₁-C₅ hydrocarbyl group, or -(CH₂)ₘO(CH₂)ₙ-, wherein m, n are each independently selected from 1, 2, 3.

In another preferred embodiment, the ionizable lipid has the structure represented by Formula (I-1), and in the Formula (I-1):
R₁ and R₂ are each independently selected from -(CH₂)ₙ-, wherein n is a positive integer ranging from 2 to 8;
L₁ is selected from the following functional groups: -(C=O)O-, -O(C=O)-;
L₂ is selected from the following functional groups: -(C=O)O-, -O(C=O)-;
R₃, R₄, R₅ and R₆ are each independently C₅-C₂₀ hydrocarbyl group;
R₇ is selected from C₁-C₅ hydrocarbyl group, or -(CH₂)ₘO(CH₂)ₙ-, wherein m, n are each independently selected from 1 or 2.

In another preferred embodiment, the ionizable lipid has the structure represented by Formula (I-2), and in the Formula (I-2):
R₁ and R₂ are each independently selected from -(CH₂)ₙ-, wherein n is a positive integer ranging from 2 to 8;
L₁ is absent or -O-(CH₂)ₙ-; wherein n is selected from 0, 1, 2, 3 or 4;
L₂ is selected from the following functional groups: -(C=O)O-, -O(C=O)-;
R₃ has the structure of -R₃ₐ-R_{3b}-R_{3c}-R_{3d}-R₃ₑ, R₄ has the structure of -R₄ₐ-R_{4b}-R_{4c}-R_{4d}-R_{4c}; R₅ has the structure of -R₅ₐ-R_{5b}-R_{5c}-R_{5d}-R₅ₑ;
wherein, R₃ₐ, R_{3c}, R₄ₐ, R_{4c}, R₅ₐ, R_{5c} are each independently-(CH₂)ₙ-, wherein n is selected from a positive integer ranging from 1 to 3;
R_{3b} and R_{4b} are each independently selected from the following group: -(C=O)O-, -O(C=O)-;
R_{3d}, R_{4d}, R_{5d} are each independently selected from the following group: -CH₂-, -(S-S)-;
R_{5b} is absent;
R₃ₑ, R₄ₑ, R₅ₑ are each independently C₂-C₁₅ hydrocarbyl group;
R₇ is selected from C₁-C₅ hydrocarbyl group, or -(CH₂)ₘO(CH₂)ₙ-, wherein m, n are each independently selected from 1 or 2.

In another preferred embodiment, the ionizable lipid has the structure represented by Formula (I-1), and in the Formula (I-1):
R₁ and R₂ are each independently selected from -(CH₂)ₙ-, wherein n a positive integer ranging from 2 to 8;
L₁ is selected from the following functional groups: -NH(C=O)-, -(C=O)NH-;
L₂ is selected from the following functional groups: -NH(C=O)-, -(C=O)NH-;
R₃, R₄, R₅ and R₆ are each independently C₅-C₂₀ hydrocarbyl group;
R₇ is selected from C₁-C₅ hydrocarbyl group, or -(CH₂)ₘO(CH₂)ₙ-, wherein m, n are each independently selected from 1 or 2.

In another preferred embodiment, the ionizable lipid has the structure represented by Formula (I-2), and in the Formula (I-2):
R₁ and R₂ are each independently selected from -(CH₂)ₙ-, wherein n is a positive integer ranging from 2 to 8;
L₁ is selected from the following functional groups: -(C=O)S-, -S(C=O)-;
L₂ is selected from the following functional groups: -(C=O)S-, -S(C=O)-;
R₃, R₄, R₅ and R₆ are each independently C₅-C₂₀ hydrocarbyl group;
R₇ is selected from C₁-C₅ hydrocarbyl group, or -(CH₂)ₘO(CH₂)ₙ-, wherein m, n are each independently selected from 1 or 2.

In another preferred embodiment, the ionizable lipid has the structure represented by Formula (I-3), and in the Formula (I-3):
R₁ and R₂ are each independently selected from -(CH₂)ₙ-, wherein n is a positive integer ranging from 2 to 8;
L₁ is absent or -O-(CH₂)ₙ-; wherein n is selected from 0, 1, 2, 3 or 4;
L₂ is absent or -O-(CH₂)ₙ-; wherein n is selected from 0, 1, 2, 3 or 4;
R₃ has the structure of -R₃ₐ-R_{3b}-R_{3c}-R_{3d}-R₃ₑ, R₄ has the structure of -R₄ₐ-R_{4b}-R_{4c}-R_{4d}-R_{4c}; R₅ has the structure of -R₅ₐ-R_{5b}-R_{5c}-R_{5d}-R₅ₑ; R₆ has the structure of -R₆ₐ-R_{6b}-R_{6c}-R_{6d}-R₆ₑ;
wherein, R₃ₐ, R_{3c}, R₄ₐ, R_{4c}, R₅ₐ, R_{5c}, R₆ₐ, R_{6c} are each independently -(CH₂)ₙ-, wherein n is selected from a positive integer ranging from 1 to 3;
R_{3b}, R_{4b}, R_{5b} and R_{6b} are each independently selected from the following group: -(C=O)O-, -O(C=O)-, -CH(OH)-;
R_{3d}, R_{4d}, R_{5d} and R_{6d} are each independently selected from the following group: -CH₂-, -(S-S)-;
R₃ₑ, R₄ₑ, R₅ₑ and R₆ₑ are each independently C₂-C₁₅ hydrocarbyl group;
R₇ is selected from C₁-C₅ hydrocarbyl group, or -(CH₂)ₘO(CH₂)ₙ-, wherein m, n are each independently selected from 1 or 2.

In another preferred embodiment, R₃, R₄, R₅ and R₆ are each independently selected from the following group: C₅-C₁₅ alkyl, C₅-C₁₅ alkenyl, C₅-C₁₅ alkynyl;
R₇ is selected from C₁-C₅ alkyl, -(CH₂)O(CH₂)-, or -(CH₂)₂O(CH₂)₂-, wherein m, n are each independently selected from 1, 2, 3.

In another preferred embodiment, the ionizable lipid has the structure selected from Table 1 below:

**Table 1**

| Serial number | Structural formula |
|---|---|
| AXT-1 | 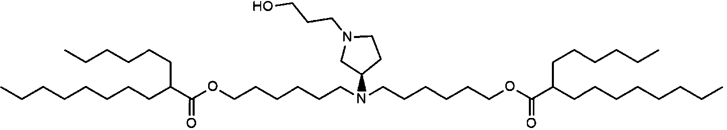 |
| AXT-2 | 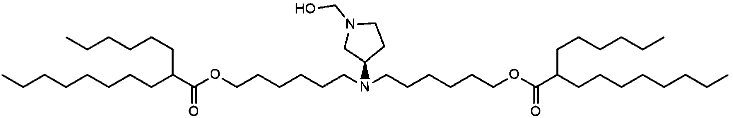 |
| AXT-3 | 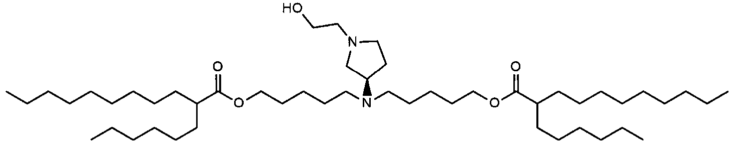 |
| AXT-4 | 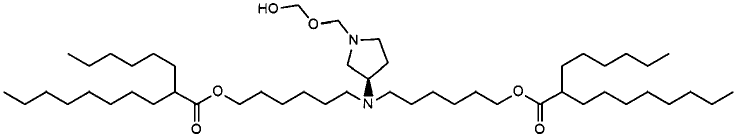 |
| AXT-5 | 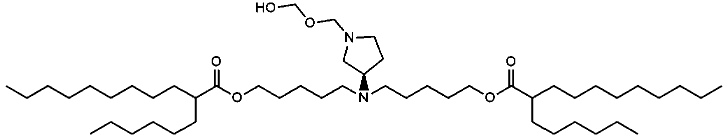 |
| AXT-6 | 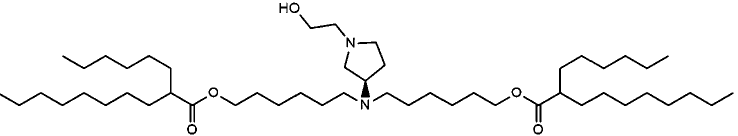 |
| AXT-7 | 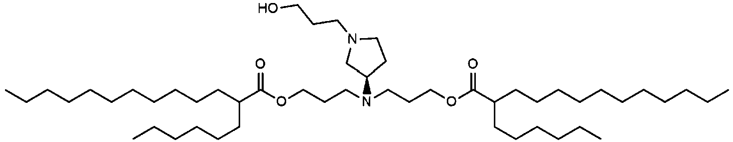 |
| AXT-8 | 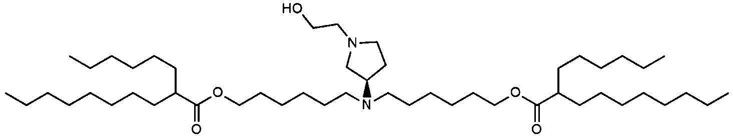 |
| AXT-9 | 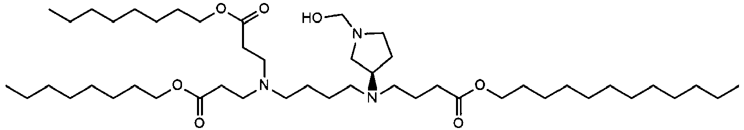 |
| AXT-10 | 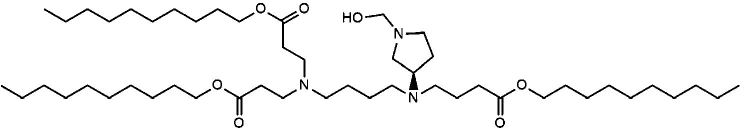 |
| AXT-11 | 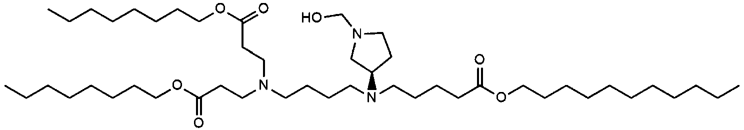 |
| AXT-12 | 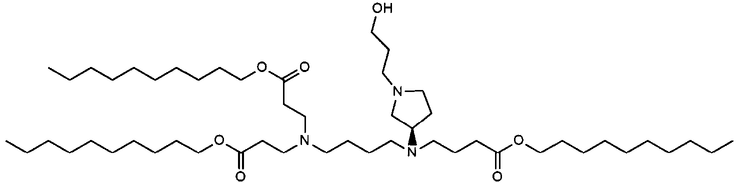 |
| AXT-13 | 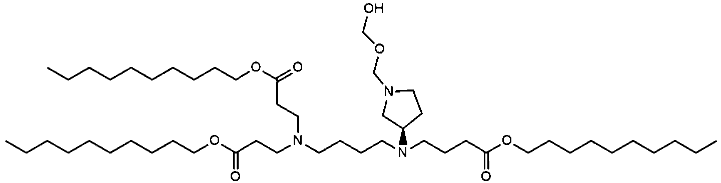 |
| AXT-14 | 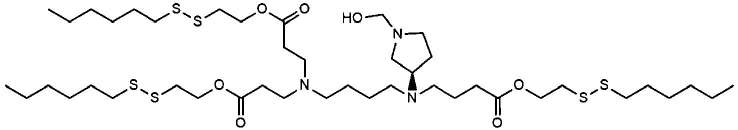 |
| AXT-15 | 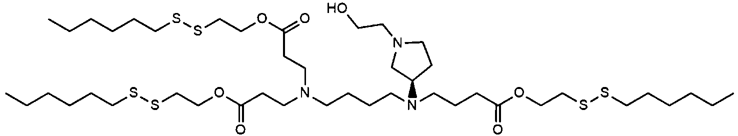 |
| AXT-16 | 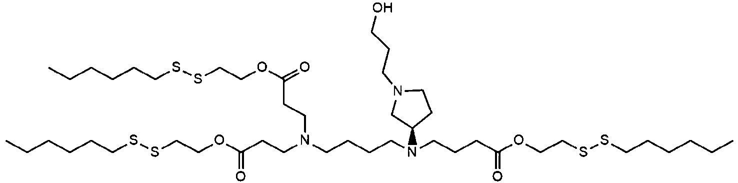 |
| AXT-17 | 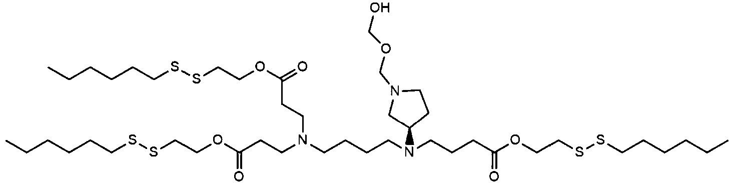 |
| AXT-18 | 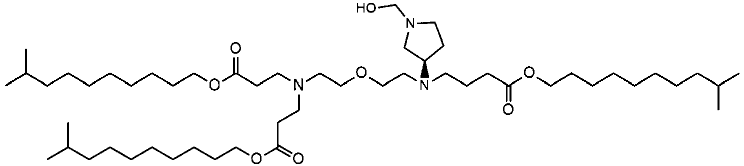 |
| AXT-19 | 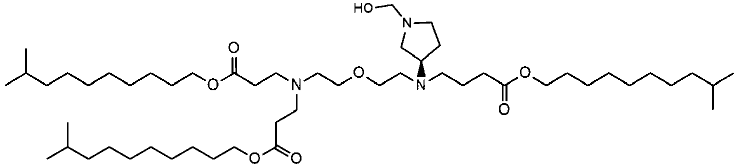 |
| AXT-20 | 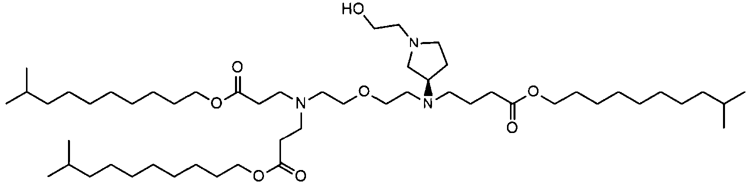 |
| AXT-21 | 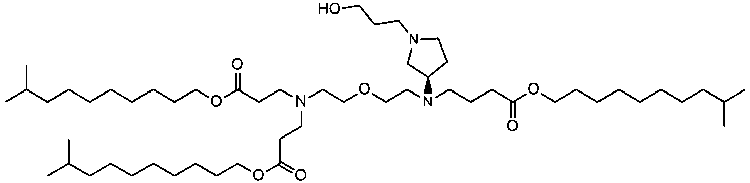 |
| AXT-22 | 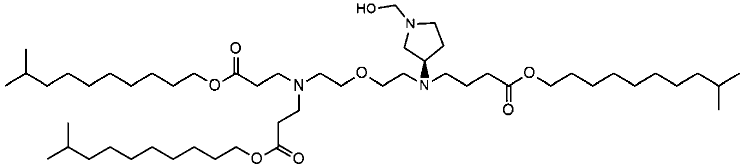 |
| AXT-23 | 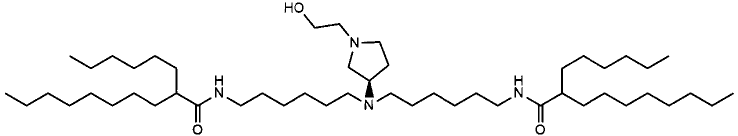 |
| AXT-24 | 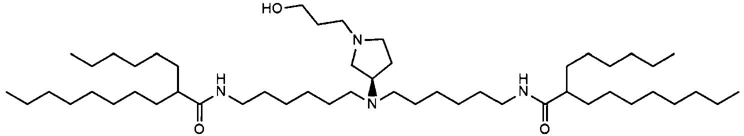 |
| AXT-25 | 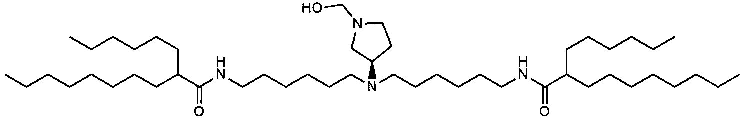 |
| AXT-26 | 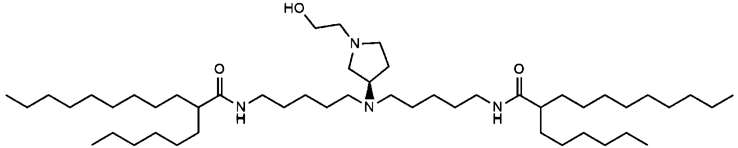 |
| AXT-27 | 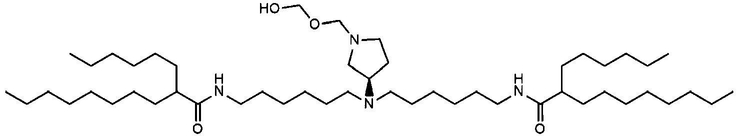 |
| AXT-28 | 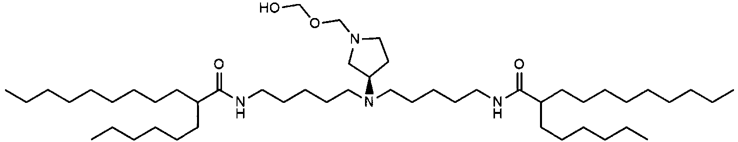 |
| AXT-29 | 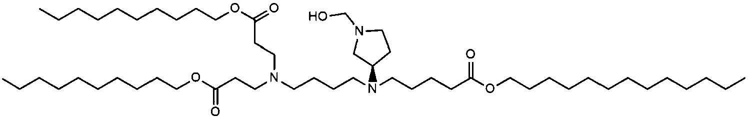 |
| AXT-30 | 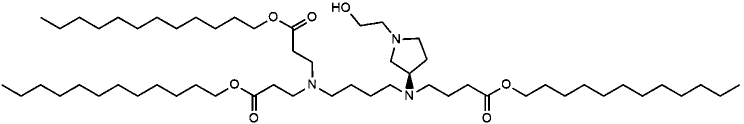 |
| AXT-31 | 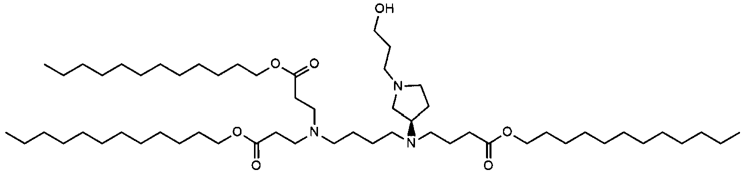 |
| AXT-32 | 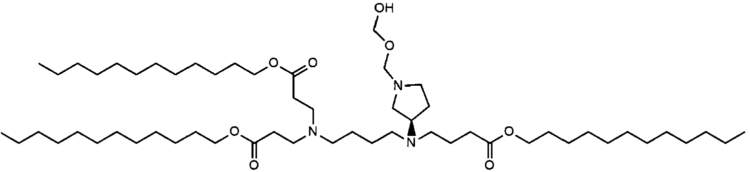 |
| AXT-33 | 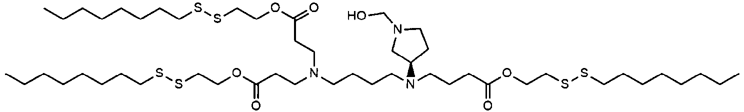 |
| AXT-34 | 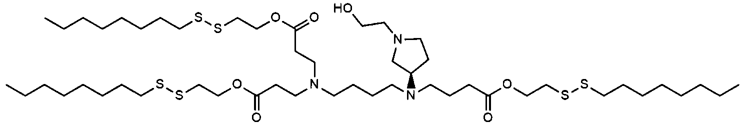 |
| AXT-35 | 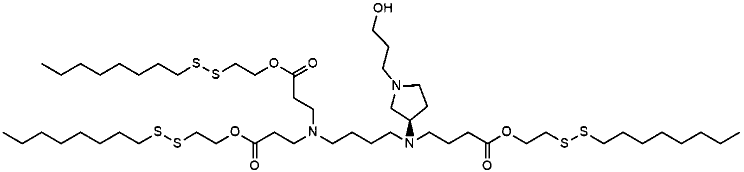 |
| AXT-36 | 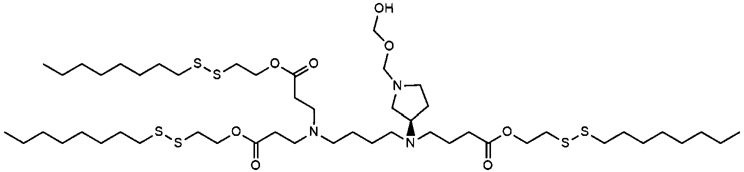 |
| AXT-37 | 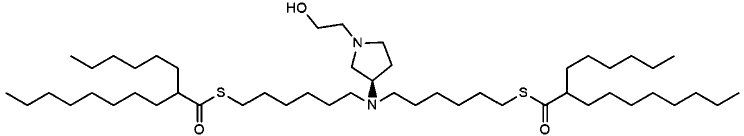 |
| AXT-38 | 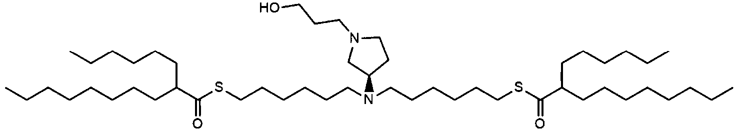 |
| AXT-39 | 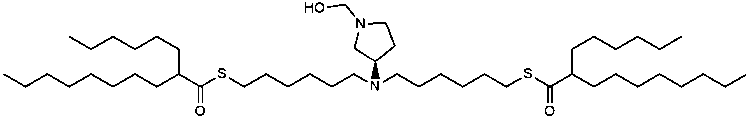 |
| AXT-40 | 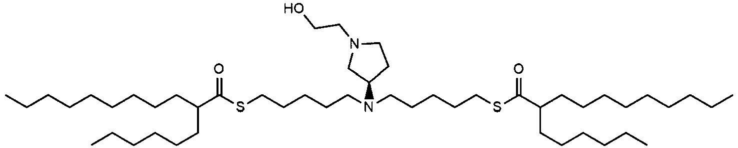 |
| AXT-41 | 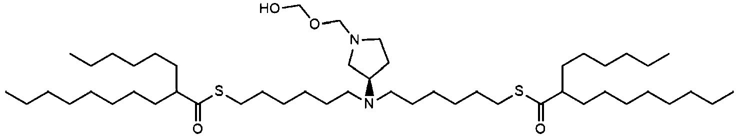 |
| AXT-42 | 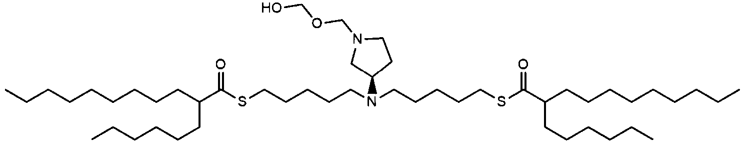 |
| AXT-43 | 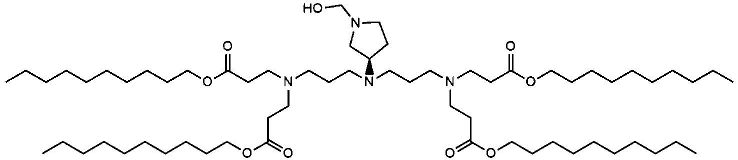 |
| AXT-44 | 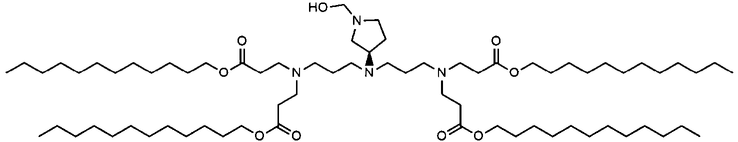 |
| AXT-45 | 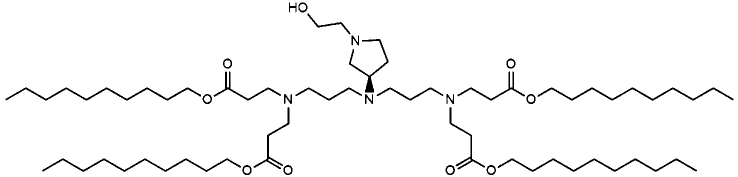 |
| AXT-46 | 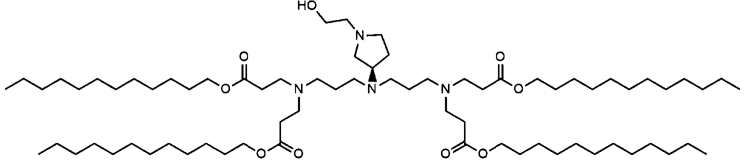 |
| AXT-47 | 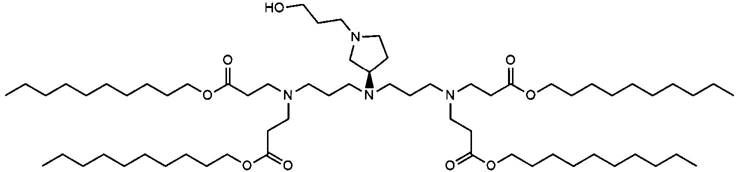 |
| AXT-48 | 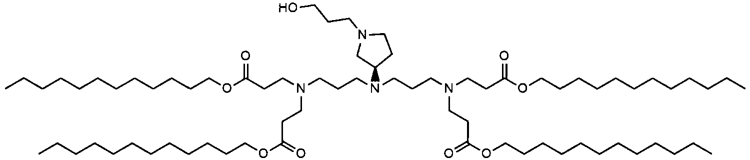 |
| AXT-49 | 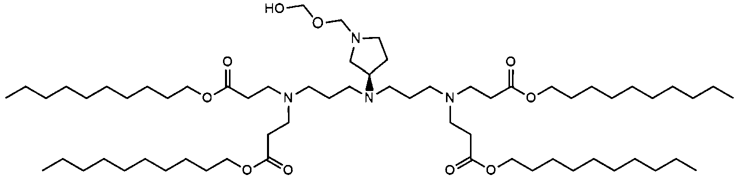 |
| AXT-50 | 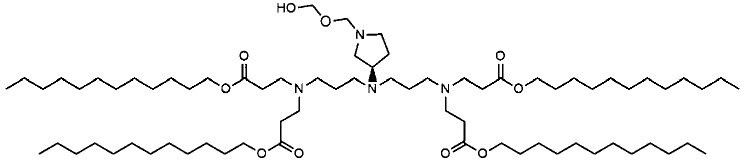 |
| AXT-51 | 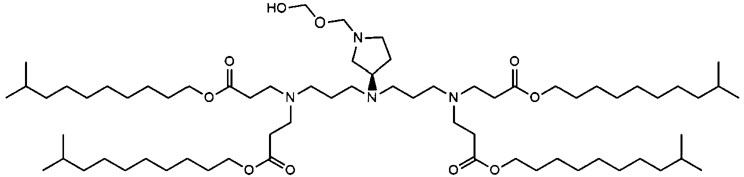 |
| AXT-52 | 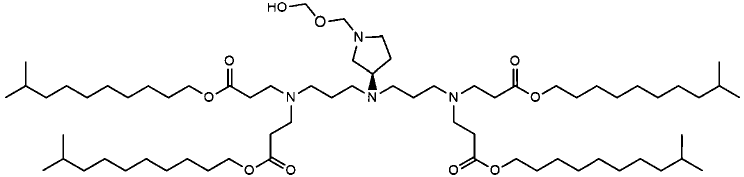 |
| AXT-53 | 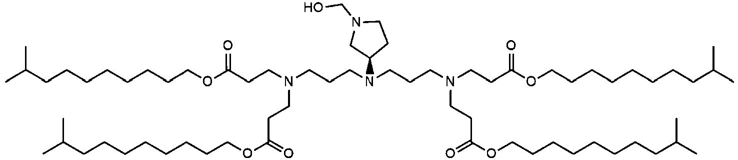 |
| AXT-54 | 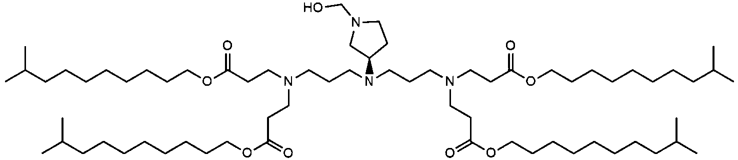 |
| AXT-55 | 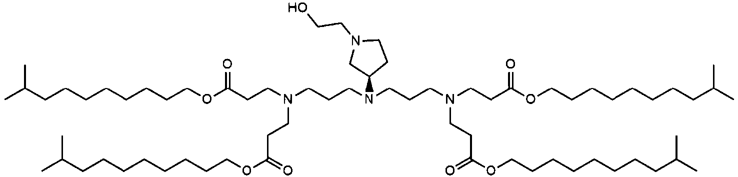 |
| AXT-56 | 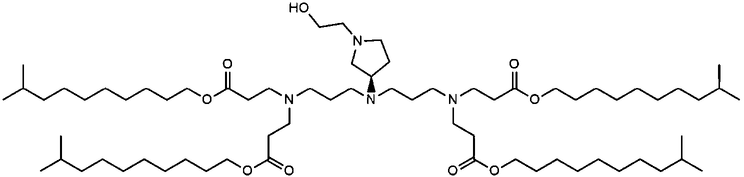 |
| AXT-57 | 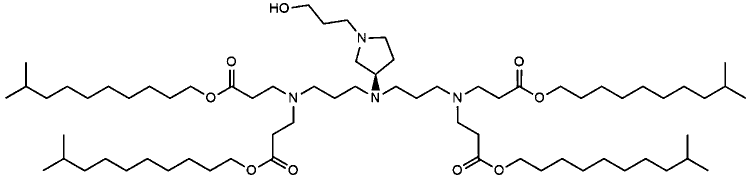 |
| AXT-58 | 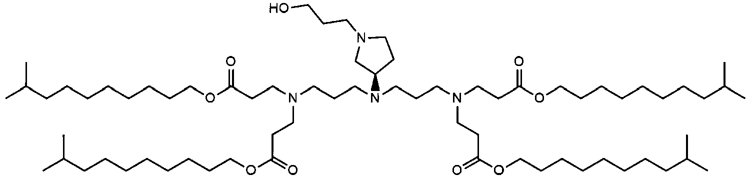 |
| AXT-59 | 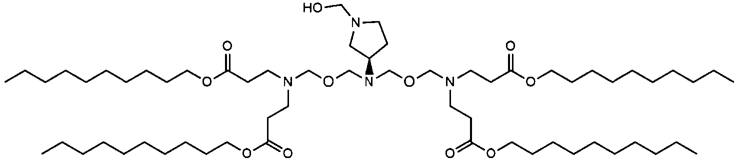 |
| AXT-60 | 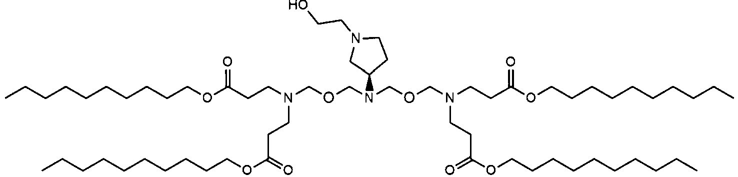 |
| AXT-61 | 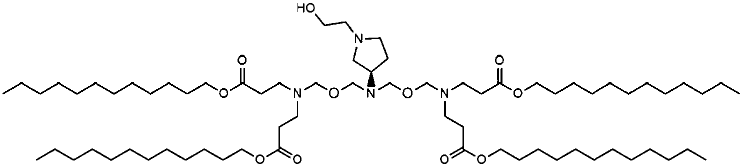 |
| AXT-62 | 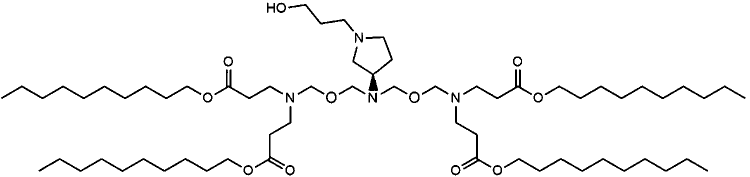 |
| AXT-63 | 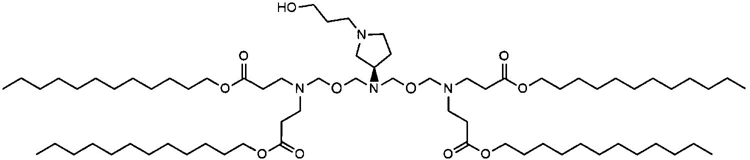 |
| AXT-64 | 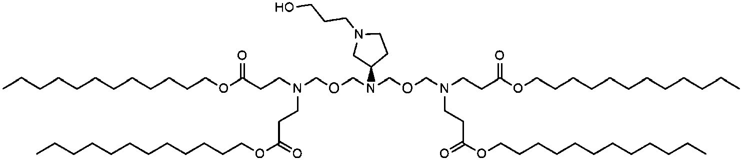 |
| AXT-65 | 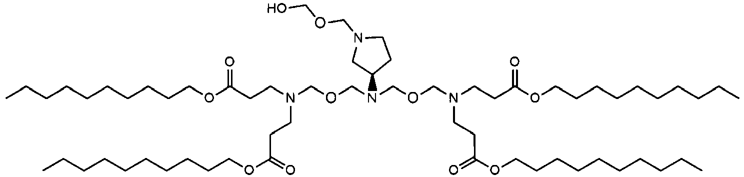 |
| AXT-66 | 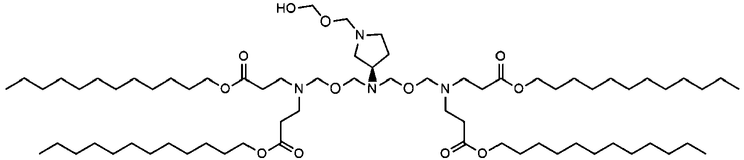 |
| AXT-67 | 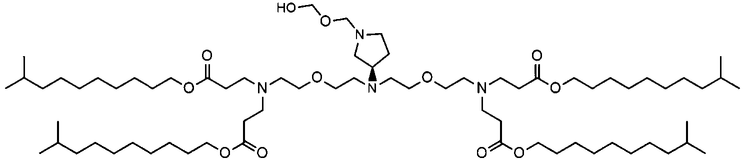 |
| AXT-68 | 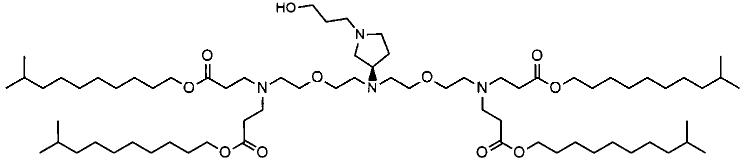 |
| AXT-69 | 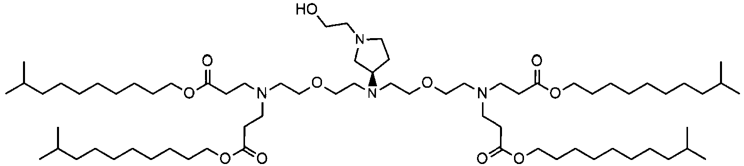 |
| AXT-70 | 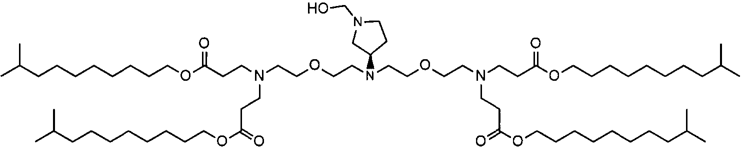 |
| AXT-71 | 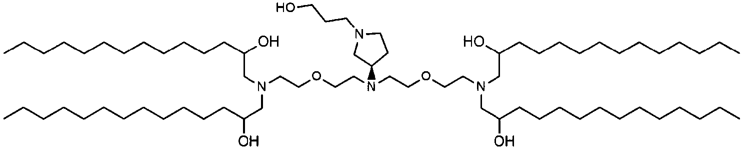 |
| AXT-72 | 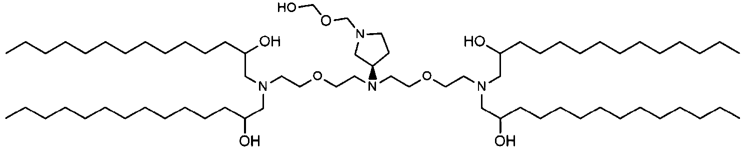 |
| AXT-73 | 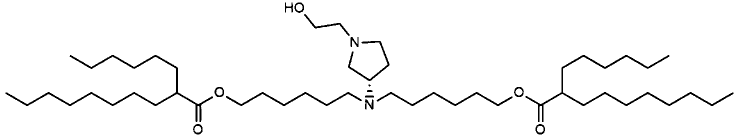 |
| AXT-74 | 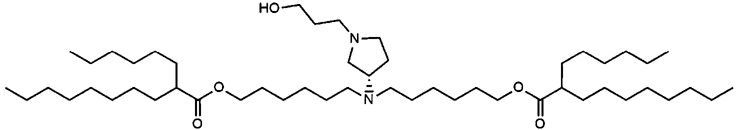 |
| AXT-75 | 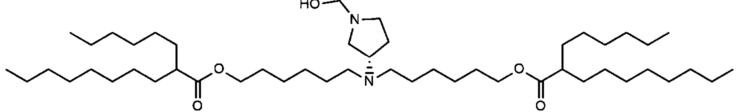 |
| AXT-76 | 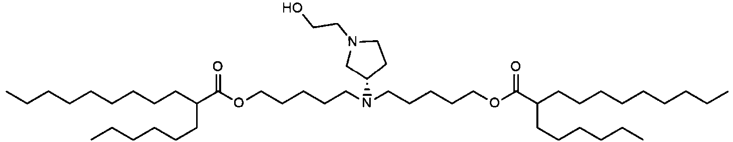 |
| AXT-77 | 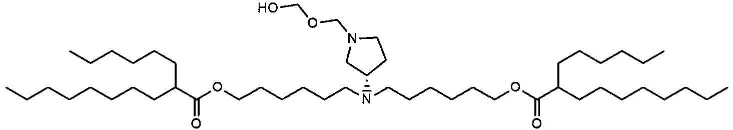 |
| AXT-78 | 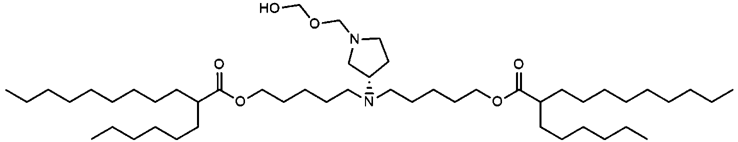 |
| AXT-79 | 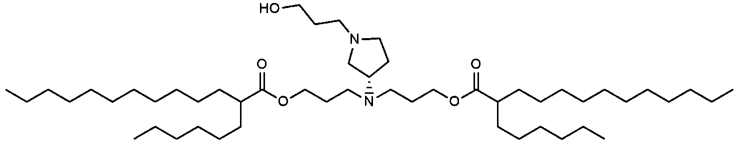 |
| AXT-80 | 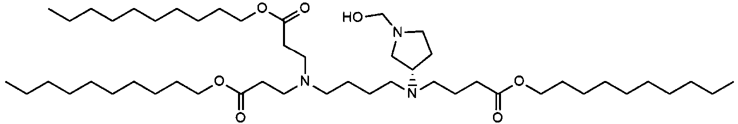 |
| AXT-81 | 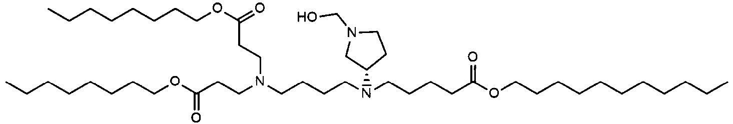 |
| AXT-82 | 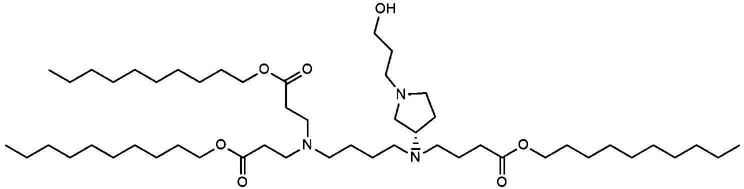 |
| AXT-83 | 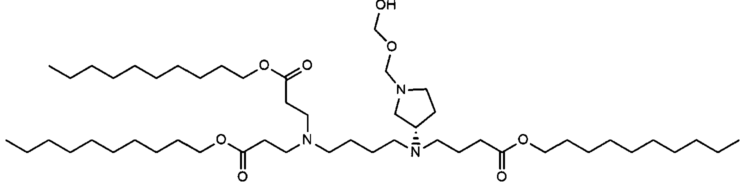 |
| AXT-84 | 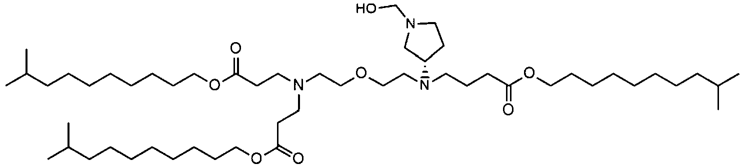 |
| AXT-85 | 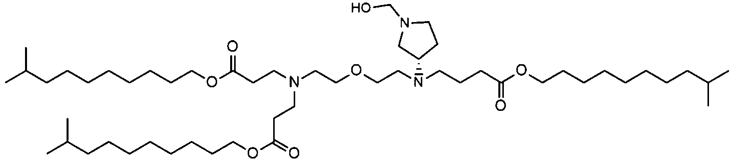 |
| AXT-86 | 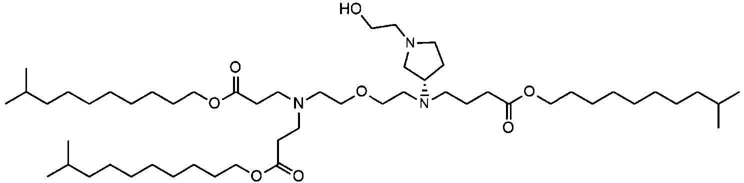 |
| AXT-87 | 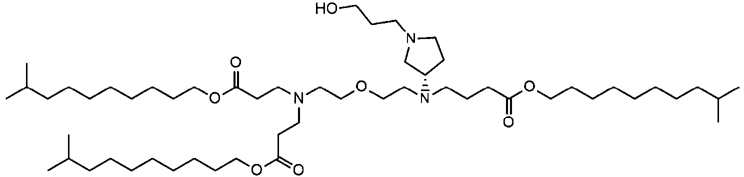 |
| AXT-88 | 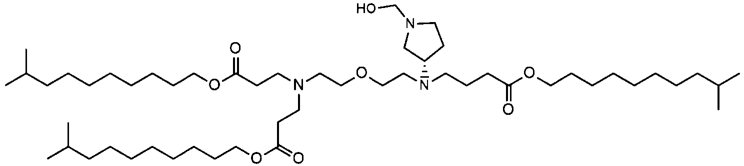 |
| AXT-89 | 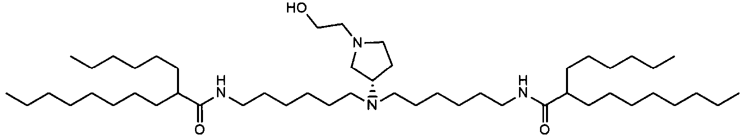 |
| AXT-90 | 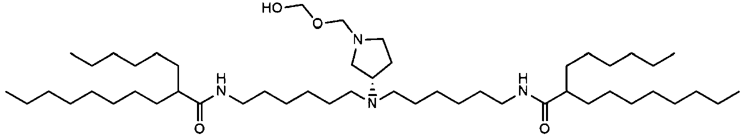 |
| AXT-91 | 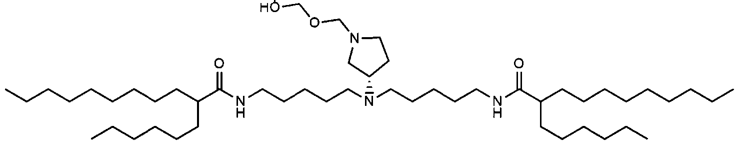 |
| AXT-92 | 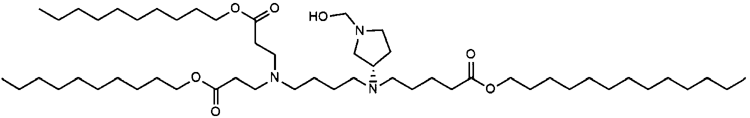 |
| AXT-93 | 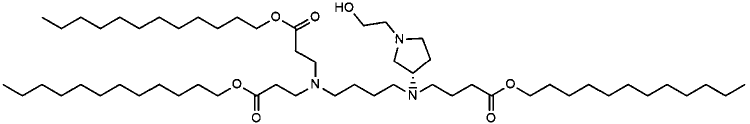 |
| AXT-94 | 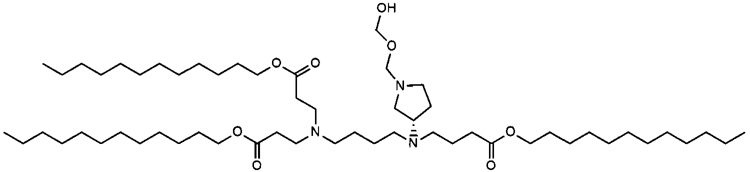 |
| AXT-95 | 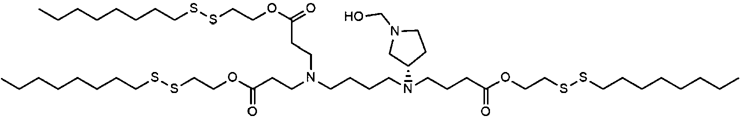 |
| AXT-96 | 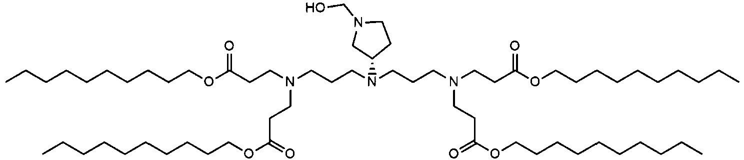 |
| AXT-97 | 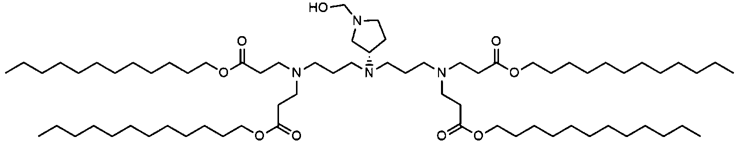 |
| AXT-98 | 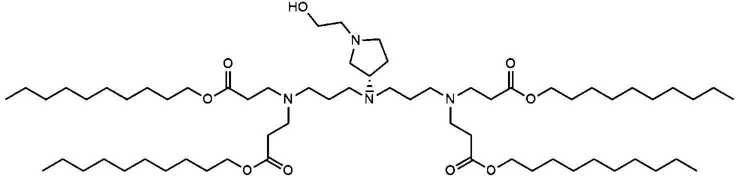 |
| AXT-99 | 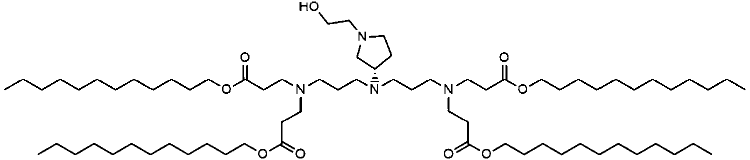 |
| AXT-100 | 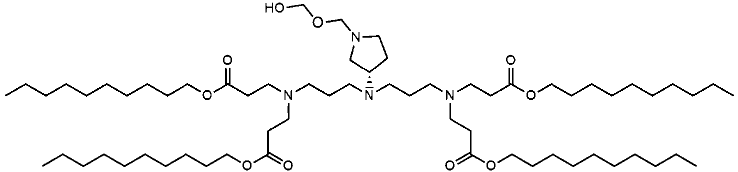 |
| AXT-101 | 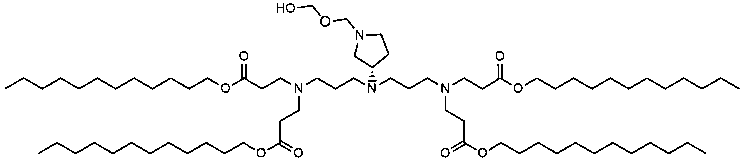 |
| AXT-102 | 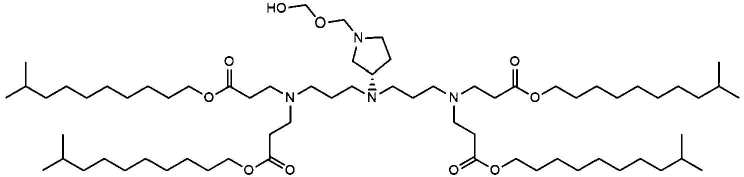 |
| AXT-103 | 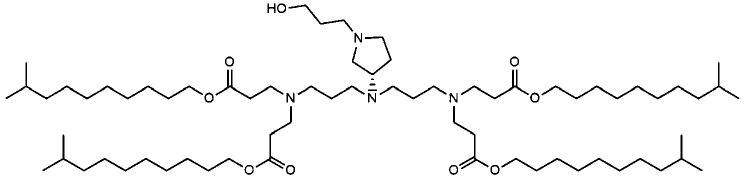 |
| AXT-104 | 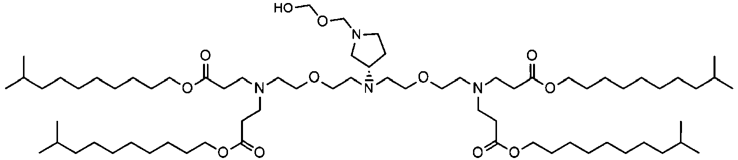 |
| AXT-105 | 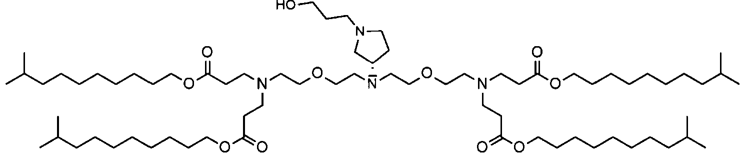 |
| AXT-106 | 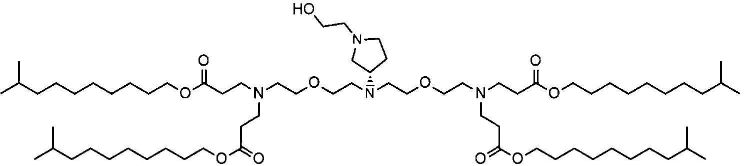 |
| AXT-107 | 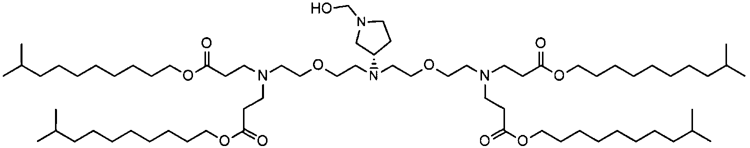 |
| AXT-108 | 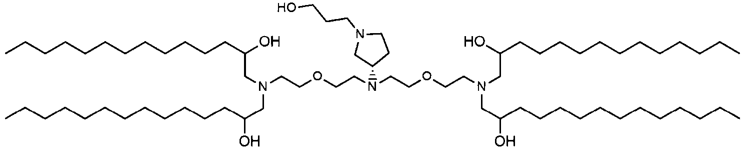 |
| AXT-109 | 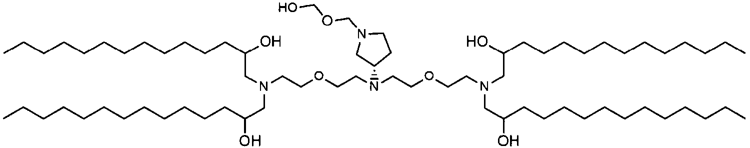 |
| AXT-110 | 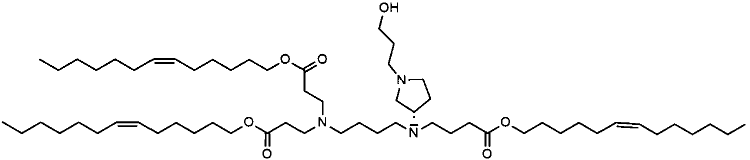 |
| AXT-111 | 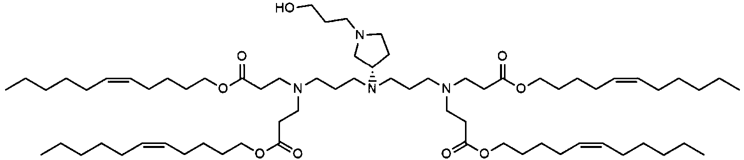 |
| AXT-112 | 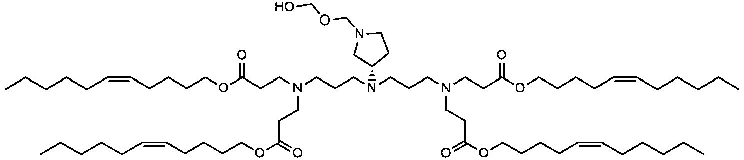 |
| AXT-113 | 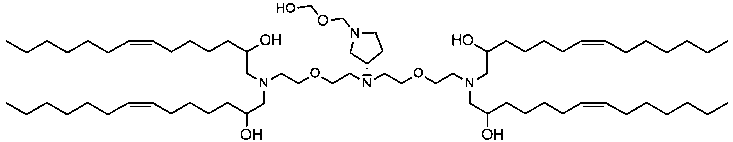 |
| AXT-114 | 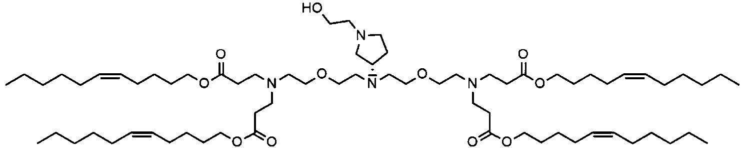 . |

In another preferred embodiment, R₃, R₄, R₅ and R₆ are each independently C₅-C₁₅ alkyl.

In another preferred embodiment, the ionizable lipid has the structure represented by Formula (I-1) below: 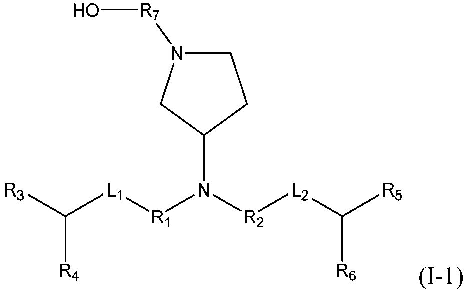 wherein,
R₁ and R₂ are each independently selected from -(CH₂)ₙ-, wherein n is a positive integer ranging from 4 to 8;
L₁ is selected from the following functional groups: -O-, -(C=O)O-, -O(C=O)-, -CH(OH)-;
L₂ is selected from the following functional groups: -O-, -(C=O)O-, -O(C=O)-, -CH(OH)-;
R₃ has the structure of -R₃ₐ-R_{3b}-R_{3c}-R_{3d}-R₃ₑ, R₄ has the structure of -R₄ₐ-R_{4b}-R_{4c}-R_{4d}-R₄ₑ; R₅ has the structure of -R₅ₐ-R_{5b}-R_{5c}-R_{5d}-R₅ₑ, R₆ has the structure of -R₆ₐ-R_{6b}-R_{6c}-R_{6d}-R₆ₑ;
wherein, R₃ₐ, R_{3c}, R₄ₐ, R_{4c}, R₅ₐ, R_{5c}, R₆ₐ and R_{6c} are each independently -(CH₂)ₙ-, wherein n is selected from a positive integer ranging from 1 to 14; and R₃, R₄, R₅ and R₆ each independently have 4-20 CH₂ structural moieties;
R_{3b}, R_{3d}, R_{4b}, R_{4d}, R_{5b}, R_{5d}, R_{6b} and R_{6d} are each independently absent or selected from the following functional groups: -(C=O)O-, -O(C=O)-, -(S-S)-, -(C=C)-(CH₂)-(C=C)-, -CH(OH)-;
R₃ₑ, R₄ₑ, R₅ₑ, R₆ₑ are each independently C₂-C₂₀ hydrocarbyl group;
R₇ is C₁-C₃ hydrocarbyl group, or -(CH₂)₂-O-(CH₂)₂-.

In another preferred embodiment, the ionizable lipid has the structure represented by the Formula below:

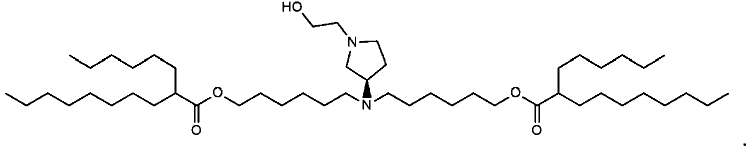

In a second aspect of the present disclosure, provides a method for preparing the ionizable lipid, or pharmaceutically acceptable salt, tautomer or stereoisomer thereof according to the first aspect of the present disclosure, the method comprises: method I, method II and method III;
wherein, method I comprises the following steps:
(S1) under the protection of an inert gas, compounds A1 and A2 are reacted to obtain compound A3;
(S2) under the protection of an inert gas, the tert-butoxycarbonyl (Boc) group of A3 is removed to obtain A4;
(S3) under the protection of an inert gas, A4 is reacted with A5 or A6 to obtain A7 (i.e., the compound represented by (I-1)); 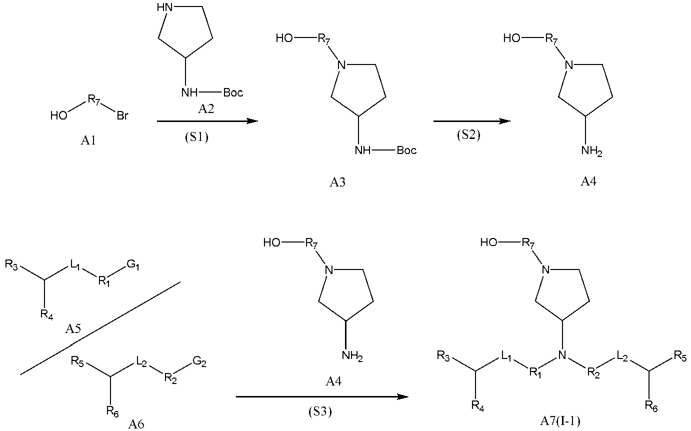
wherein, R₁ and R₂ are each independently selected from -(CH₂)ₙ-, wherein n is a positive integer ranging from 1 to 14;
G₁ and G₂ are each independently selected from an active functional group, preferably selected from a carbonyl group, halogen (fluorine, chlorine, bromine, iodine, etc.), an ethylene oxide group, etc.
L₁ has the structure of-(L₁ₐ-L_{1b})- from right to left, or is absent, wherein, L₁ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, preferably selected from -O-, -(C=O)O-, -O(C=O)-, -CH(OH)-; L_{1b} is -(CH₂)ₙ-, wherein n is selected from 0, 1, 2, 3 or 4;
L₂ has the structure of -(L₂ₐ-L_{2b})- from left to right, or is absent; wherein, L₂ₐ is selected from the following functional groups: O, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, preferably selected from O, -(C=O)O-, -O(C=O)-, -CH(OH)-; L_{2b} is -(CH₂)ₙ-, wherein n is selected from 0, 1, 2, 3 or 4;
R₃, R₄, R₅ and R₆ are each independently C₂-C₂₀ hydrocarbyl group.
R₇ is selected from C₁-C₅ hydrocarbyl group, or -(CH₂)ₘO(CH₂)ₙ-, wherein m, n are each independently selected from 1, 2, 3;
method II comprises the following steps:
   (D1) under the protection of an inert gas, compound B1 is reacted with B2 to obtain compound B3;
   (D2) under the protection of an inert gas, compound B3 is reacted with B4 to obtain compound B5;
   (D3) under the protection of an inert gas, compound B5 is reacted with B6 to obtain B7, i.e., the compound represented by (I-2); 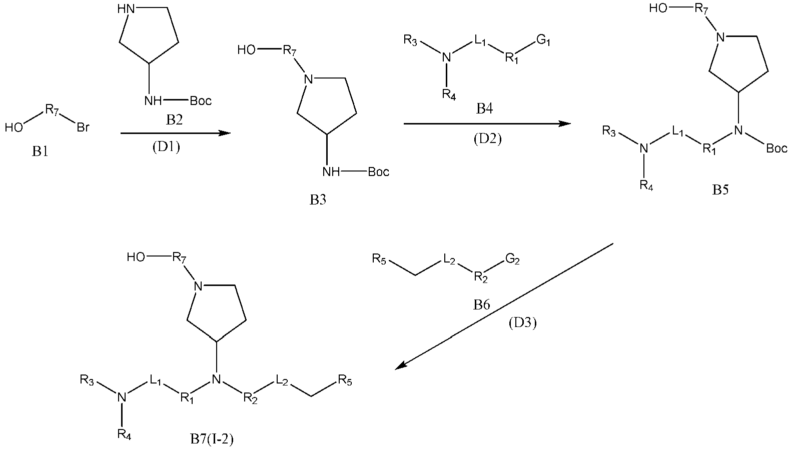
   wherein, R₁ and R₂ are each independently selected from -(CH₂)ₙ-, wherein n is a positive integer ranging from 1 to 14;
   G₁ and G₂ are each independently selected from an active functional group, preferably selected from a carbonyl group, halogen (fluorine, chlorine, bromine, iodine, etc.), an ethylene oxide group, etc.
   L₁ has the structure of -(L₁ₐ-L_{1b})- from right to left, or is absent, wherein, L₁ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, preferably selected from -O-, -(C=O)O-, -O(C=O)-, -CH(OH)-; L_{1b} is -(CH₂)ₙ-, wherein n is selected from 0, 1, 2, 3 or 4;
   L₂ has the structure of -(L₂ₐ-L_{2b})- from left to right, or is absent; wherein, L₂ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, preferably selected from -O-, -(C=O)O-, -O(C=O)-, -CH(OH)-; L_{2b} is -(CH₂)ₙ-, wherein n is selected from 0, 1, 2, 3 or 4;
   R₃ has the structure of -R₃ₐ-R_{3b}-R_{3c}-R_{3d}-R₃ₑ, R₄ has the structure of -R₄ₐ-R_{4b}-R_{4c}-R_{4d}-R₄ₑ; R₅ has the structure of -R₅ₐ-R₅₆-R_{5c}-R_{5d}-R₅ₑ;
   wherein, R₃ₐ, R_{3c}, R₄ₐ, R_{4c}, R₅ₐ, R_{5c} are each independently -(CH₂)ₙ-, wherein n is selected from a positive integer ranging from 1 to 14;
   R_{3b}, R_{3d}, R_{4b}, R_{4d}, R_{5b}, R_{5d} are each independently absent or selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, -(C=C)-(CH₂)-(C=C)-, -(C=C)-, -CH₂-, -(C=C)-, preferably -(C=O)O-, -O(C=O)-, -(S-S)-, -(C=C)-(CH₂)-(C=C)-, -CH(OH)-;
   R₃ₑ, R₄ₑ, R₅ₑ are each independently C₂-C₂₀ hydrocarbyl group;
   R₆ is H;
   R₇ is selected from C₁-C₅ hydrocarbyl group, or -(CH₂)ₘO(CH₂)ₙ-, wherein m, n are each independently selected from 1, 2, 3;
   method III comprises the following steps:
      (M1) under the protection of an inert gas, compounds C1 and C2 are reacted to obtain C3;
      (M2) under the protection of an inert gas, compound C3 undergoes de-Boc deprotection to obtain compound C4;
      (M3) under the protection of an inert gas, compound C4 is reacted with C5 or C6 to obtain compound C7, i.e., the compound represented by Formula (I-3); 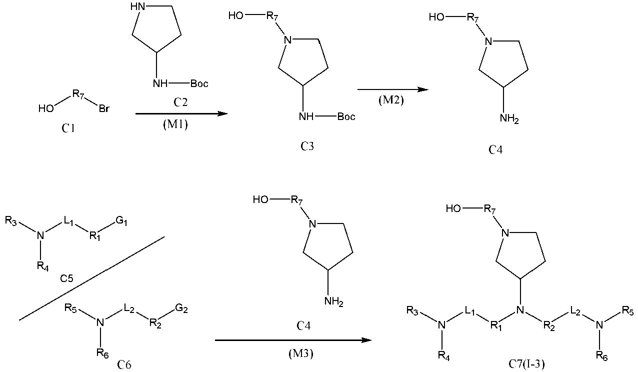
      wherein, R₁ and R₂ are each independently selected from -(CH₂)ₙ-, wherein n is a positive integer ranging from 1 to 14;
      G₁ and G₂ are each independently selected from an active functional group, preferably selected from a carbonyl group, halogen (fluorine, chlorine, bromine, iodine, etc.), an ethylene oxide group, etc.
      L₁ has the structure of -(L₁ₐ-L_{1b})- from right to left, or is absent, wherein, L₁ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, preferably selected from -O-, -(C=O)O-, -O(C=O)-, -CH(OH)-; L_{1b} is -(CH₂)ₙ-, wherein n is selected from 0, 1, 2, 3 or 4;
      L₂ has the structure of -(L₂ₐ-L_{2b})- from left to right, or is absent; wherein, L₂ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, preferably selected from -O-, -(C=O)O-, -O(C=O)-, -CH(OH)-; L_{2b} is -(CH₂)ₙ-, wherein n is selected from 0, 1, 2, 3 or 4;
      R₃ has the structure of -R₃ₐ-R_{3b}-R_{3c}-R_{3d}-R₃ₑ, R₄ has the structure of -R₄ₐ-R_{4b}-R_{4c}-R_{4d}-R₄ₑ; R₅ has the structure of -R₅ₐ-R_{5b}-R_{5c}-R_{5d}-R₅ₑ, R₆ has the structure of -R₆ₐ-R_{6b}-R_{6c}-R_{6d}-R₆ₑ;
      wherein, R₃ₐ, R_{3c}, R₄ₐ, R_{4c}, R₅ₐ, R_{5c}, R₆ₐ and R_{6c} are each independently -(CH₂)ₙ-, wherein n is selected from a positive integer ranging from 1 to 14;
      R_{3b}, R_{3d}, R_{4b}, R_{4d}, R_{5b}, R_{5d}, R_{6b} and R_{6d} are each independently absent or selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, -(C=C)-(CH₂)-(C=C)-, -(C=C)-, -CH₂-, -(C=C)-, preferably -(C=O)O-, -O(C=O)-, -(S-S)-, -(C=C)-(CH₂)-(C=C)-, -CH(OH)-;
      R₃ₑ, R₄ₑ, R₅ₑ, R₆ₑ are each independently C₂-C₂₀ hydrocarbyl group;
      R₇ is selected from C₁-C₅ hydrocarbyl group, or -(CH₂)ₘO(CH₂)ₙ-, wherein m, n are each independently selected from 1, 2, 3.

In a third aspect of the present disclosure, provides a lipid nanoparticle (LNP), the lipid nanoparticle comprises the ionizable lipid, or pharmaceutically acceptable salt, tautomer or stereoisomer thereof according to the first aspect of the present disclosure.

In another preferred embodiment, the lipid nanoparticle further comprises an auxiliary lipid.

In another preferred embodiment, in the lipid nanoparticle, the content of the ionizable lipid accounts for 30-65 mol% of the total lipid content.

In another preferred embodiment, the auxiliary lipid includes helper phospholipids, sterols, polymer-conjugated lipids, or a combination thereof.

In another preferred embodiment, the auxiliary lipid is a combination of helper phospholipids, sterols and polymer-conjugated lipids.

In another preferred embodiment, the helper phospholipid is preferably selected from:
1,2-distearoyl-sn-glycero-3-phosphocholine (DSPC),
1,2-dioleoyl-sn-glycero-3-phosphoethanolamine (DOPE), dioleoylphosphatidylcholine (DOPC), 1,2-dioleoyl-sn-glycero-3-phosphocholine,
1,2-dipalmitoyl-sn-glycero-3-phosphocholine,
1,2-dipalmitoyl-sn-glycero-3-phosphoethanolamine,
1,2-dimyristoyl-sn-glycero-3-phosphoethanolamine, sodium
1,2-dioleoyl-sn-glycero-3-phospho-rac-(1-glycerol), 1,2-dipalmitoylphosphatidylglycerol,
1-palmitoyl-2-oleoylphosphatidylcholine, 1-palmitoyl-2-oleoylphosphoethanolamine,
distearoylphosphoethanolamine, 1-stearoyl-2-oleoylphosphatidylcholine,
1-stearoyl-2-oleoylphosphoethanolamine, or a combination thereof.

In another preferred embodiment, the sterol includes cholesterol or cholesterol derivatives.

In another preferred embodiment, the polymer-conjugated lipid is pegylated (PEG) lipids.

In another preferred embodiment, the pegylated lipid is preferably selected from the following group: DMG-PEG2000, DSPE-PEG2000, DSG-PEG2000, DSPE-PEG-Mannose, DMG-PEG2000-(polypeptides, proteins, amino acids, vitamins and the other active substances), or a combination thereof.

In another preferred embodiment, the lipid nanoparticle comprises an ionizable lipid, DSPC, cholesterol and DMG-PEG2000, wherein the molar ratio of the ionizable lipid:DSPC:cholesterol:DMG-PEG2000 is (30-65):(5-30):(30-55):(1-5), preferably (40-50):(10-15):(38-45):(1.5-2).

In another preferred embodiment, the lipid nanoparticle further comprises a bioactive substance encapsulated in the lipid nanoparticle.

In another preferred embodiment, the bioactive substance is selected from the following group: nucleic acids, proteins, polypeptides, small molecules, or a combination thereof.

In another preferred embodiment, the nucleic acid includes DNA, plasmids, messenger RNA (mRNA), small interfering RNA (siRNA), antisense oligonucleotides, small RNAs, ribosomal RNAs, microRNAs and transfer RNAs, preferably is mRNA.

In a fourth aspect of the present disclosure, provides a lipid nanoparticle pharmaceutical formulation, the lipid nanoparticle pharmaceutical formulation comprises:
i) the lipid nanoparticle according to the third aspect of the present disclosure;
ii) a biologically active substance encapsulated in the lipid nanoparticle; and
iii) a pharmaceutically acceptable carrier.

In another preferred embodiment, the bioactive substance is selected from the following group: nucleic acids, proteins, polypeptides, small molecules, or a combination thereof.

In another preferred embodiment, the nucleic acid includes DNA, plasmids, messenger RNA (mRNA), small interfering RNA (siRNA), antisense oligonucleotides, small RNAs, ribosomal RNAs, microRNAs and transfer RNAs, preferably is mRNA.

In another preferred embodiment, the bioactive substance is a nucleic acid, and in the lipid nanoparticle pharmaceutical formulation, the molar ratio of ionizable N atoms in the ionizable lipid molecules to phosphate groups in the nucleic acid molecules is (2~10):1, further preferably (4~8):1.

In another preferred embodiment, the hydrated particle size of the lipid nanoparticle pharmaceutical formulation is 50-200 nm, more preferably 70-150 nm, and most preferably 75-110 nm.

In another preferred embodiment, the lipid nanoparticle pharmaceutical formulation can be used for treating and/or preventing a tumor, an infectious disease and a rare disease.

In another preferred embodiment, the dosage form of the lipid nanoparticle pharmaceutical formulation is selected from the following group: injections, lyophilized preparations, aerosol inhalants, and topical formulations.

In another preferred embodiment, the lipid nanoparticle pharmaceutical formulation is administered *via* injection, i.e., intravenous, intramuscular, intradermal, subcutaneous, intrathecal, intraduodenal or intraperitoneal injection.

In another preferred embodiment, the lipid nanoparticle pharmaceutical formulation is administered *via* inhalation, e.g., intranasal administration.

In another preferred embodiment, the lipid nanoparticle pharmaceutical formulation is administered transdermally, e.g., *via* transdermal application or iontophoresis.

In a fifth aspect of the present disclosure, provides a method for preparing the lipid nanoparticle pharmaceutical formulation according to the fourth aspect of the present disclosure, the method comprises:
(a) mixing the ionizable lipid, or pharmaceutically acceptable salt, tautomer or stereoisomer thereof according to the first aspect of the present disclosure, and optional auxiliary lipid with an organic solvent to obtain a lipid organic phase.
(b) mixing a biologically active substance with an aqueous solvent to obtain an aqueous phase containing the biologically active substance;
(c) mixing the lipid organic phase in step (a) with the aqueous phase in step (b) to obtain the lipid nanoparticle pharmaceutical formulation.

In another preferred embodiment, the organic solvents include ethanol, methanol, isopropanol, acetonitrile, dimethylformamide, dimethyl sulfoxide, dioxane, tetrahydrofuran or a combination thereof.

In another preferred embodiment, the aqueous solvent is a buffer solution.

In another preferred embodiment, the aqueous solvent is a buffer solution with a pH range of 3-7.

In another preferred embodiment, the acidic buffer solution is a citrate buffer solution with a pH of 4.0.

In another preferred embodiment, the volume ratio of the lipid organic phase to the aqueous phase containing the bioactive substance is 1:(2-5), more preferably 1:(3-4).

In another preferred embodiment, in step (c), the lipid organic phase and the aqueous phase are mixed *via* a microfluidic chip.

In another preferred embodiment, the method further comprises step (d): purifying, concentrating and sterilizing by filtration the lipid nanoparticle pharmaceutical formulation obtained in step (c).

In a sixth aspect of the present disclosure, provides a use of the ionizable lipid, or pharmaceutically acceptable salt, tautomer or stereoisomer thereof according to the first aspect of the present disclosure in the manufacture of a drug delivery system.

In another preferred embodiment, the delivery system includes lipid nanoparticles (LNPs), liposomes, polymer nanoparticles, etc, and is preferably used for preparing lipid nanoparticles.

In another preferred embodiment, the drug delivery system is used for delivering drugs for treating and/or preventing a tumor, an infectious disease and a rare disease.

In a seventh aspect of the present disclosure, provides a use of the lipid nanoparticle according to the third aspect of the present disclosure in the manufacture of a medicament for treating and/or preventing a tumor, an infectious disease and a rare disease.

In an eighth aspect of the present disclosure, provides a use of the ionizable lipid, or pharmaceutically acceptable salt thereof according to the first aspect of the present disclosure, the use in the manufacture of the lipid nanoparticle pharmaceutical formulation. The lipid nanoparticle pharmaceutical formulation is used for delivering a bioactive substance to the cells of a subject in need thereof.

In another preferred embodiment, the bioactive substance is selected from the following group: DNA, RNA (mRNA, tRNA, rRNA, miRNA), and natural and synthetic oligonucleotides (including antisense oligonucleotides (ASO), interfering RNA (RNAi) and small interfering RNA (siRNA)).

In another preferred embodiment, the bioactive substance is mRNA.

In another preferred embodiment, the mRNA is transfected into cells and expressed in the cells.

In another preferred embodiment, the cells are immune cells.

In another preferred embodiment, the cells include T cells, B cells, NK cells, or a combination thereof.

In another preferred embodiment, the T cells are selected from the following group: human primary T cells, JM cells, Jurkat cells, or a combination thereof.

In another preferred embodiment, the human primary T cells include helper T cells, regulatory T cells, and memory T cells.

In another preferred embodiment, the ionizable lipid has the structure represented by the Formula below:

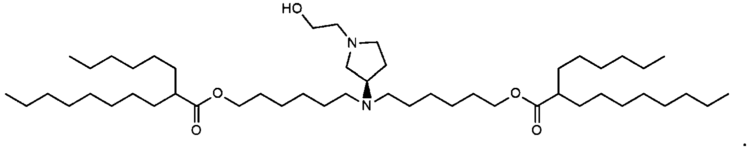

It should be understood that within the scope of the present disclosure, the aforementioned technical features of the present disclosure and the various technical features specifically described hereinafter (e.g., the examples) can be combined with each other to form new or preferred technical solutions. Due to space limitations, no further enumeration will be provided herein.

### BRIEF DESCRIPTION OF THE DRAWINGS

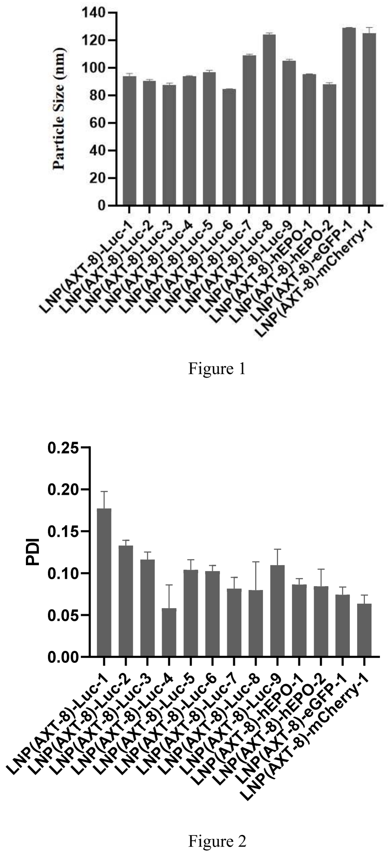

Figure 1 shows the particle size characterization results of LNP-mRNA formulations with different compositions composed of AXT-8, with the particle size ranging from 80 nm to130 nm, which demonstrates that the LNP prepared as described above have similar physicochemical properties. (Note: In the legends above, LNP(AXT-8)-Luc-1 refers to the LNP composed of molecule AXT-8 encapsulating Luc mRNA, where "1" denotes Formulation 1 among the series of LNPs encapsulating Luc mRNA, and the rest can be deduced by analogy; LNP(AXT-8)-hEPO-1 refers to the LNP composed of molecule AXT-8 encapsulating hEPO mRNA, where "1" denotes Formulation 1 among the series of LNPs encapsulating hEPO mRNA, and the rest can be deduced by analogy; LNP(AXT-8)-eGFP-1 refers to the LNP composed of molecule AXT-8 encapsulating eGFP mRNA, where "1" denotes Formulation 1 among the series of LNPs encapsulating eGFP mRNA; LNP(AXT-8)-mCherry-1 refers to the LNP composed of molecule AXT-8 encapsulating mCherry mRNA, where "1" denotes Formulation 1 among the series of LNPs encapsulating mCherry mRNA.)
Figure 2 shows the particle size distribution characterization results of LNP-mRNA formulations with different compositions composed of AXT-8, with PDI<0.2, which demonstrates that the nanoparticles composed of AXT-8 have a narrow particle size distribution.

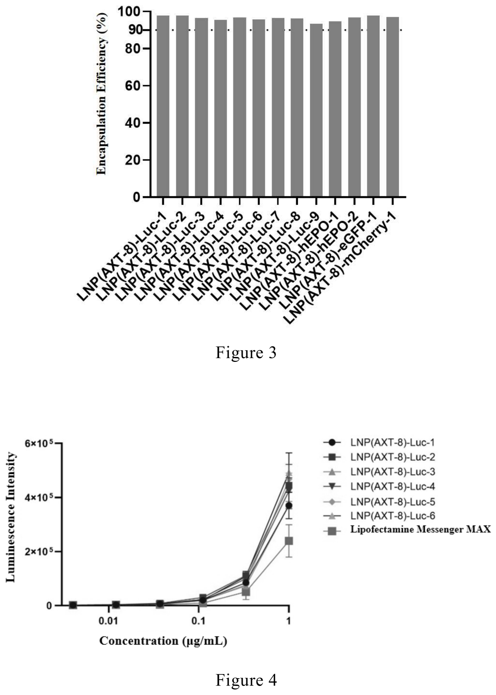

Figure 3 shows the encapsulation efficiency characterization results of LNP-mRNA formulations with different compositions composed of AXT-8, with encapsulation efficiencies > 90%, which demonstrates that the encapsulation effect is favorable.
Figure 4 shows the transfection results of LNP-mRNA with different ratios composed of AXT-8 in 293T cells. As illustrated in the figure, with the increase in mRNA concentration, the cellular expression results of multiple formulations of LNP(AXT-8)-mRNA are all superior to those of Lipofectamine.

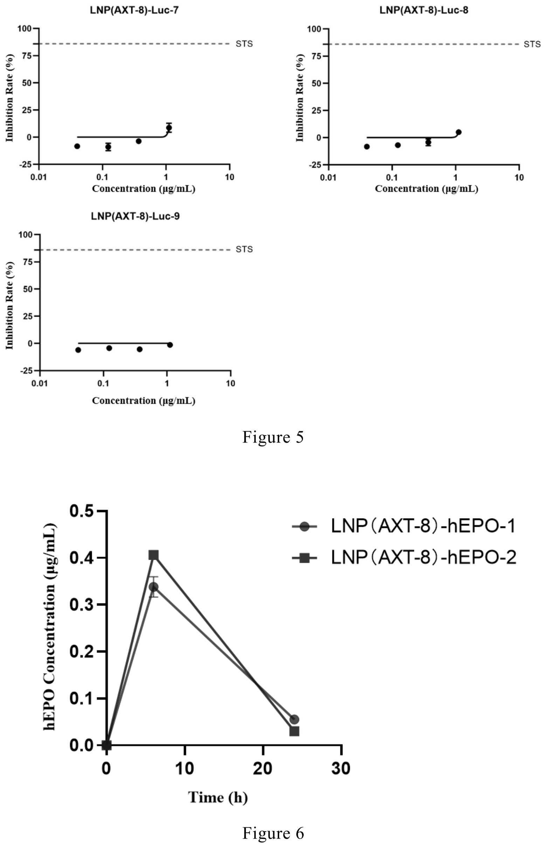

Figure 5 shows the cytotoxicity results of LNP(AXT-8)-mRNA composed of AXT-8. As illustrated in the figure, when the mRNA concentration of the LNP(AXT-8) product series is below 1 µg/mL, the cell inhibition rate thereof is nearly 0.
Figure 6 shows the *in vivo* hEPO expression results of LNP-mRNA formulations with different compositions composed of AXT-8. As illustrated in the figure, for the different formulations, the trend of *in vivo* mRNA expression increases first and then decreases, and the peak expression is achieved at approximately 6 h for all formulations.

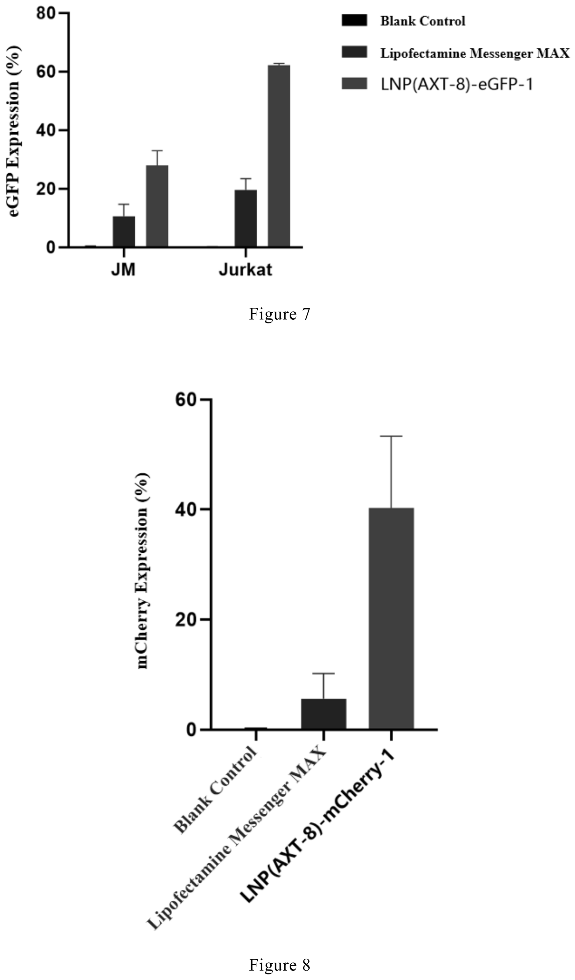

Figure 7 shows the *in vitro* transfection expression results of LNP-mRNA formulations composed of AXT-8 in JM cells and Jurkat cells. As illustrated in the figure, LNP(AXT-8)-mRNA can be successfully expressed in both JM cells and Jurkat cells, with the expression levels being superior to those of Lipofectamine Max.
Figure 8 shows the *in vitro* transfection expression results of LNP-mRNA formulations composed of AXT-8 in human primary T cells. As illustrated in the figure, LNP(AXT-8)-mRNA can be successfully expressed in human primary T cells, with the expression levels being superior to those of Lipofectamine Max.

### DETAILED EMBODIMENTS

After extensive and in-depth research was conducted by the Applicant, an ionizable lipid was unexpectedly discovered for the first time. The ionizable lipid was characterized by stable physicochemical properties and low toxicity. The drug delivery system obtained by encapsulating a drug payload (e.g., mRNA) with the ionizable lipid of the present disclosure exhibited high delivery efficiency and low toxicity. While enabling efficient delivery of the drug payload and increasing the expression level of the drug payload, the safety of the drug delivery system was improved, thereby rendering the preventive and therapeutic effects of the drug delivery system more prominent. On the basis of the aforementioned findings, the present disclosure was completed.

### Definition

To facilitate a better understanding of the present disclosure, certain terms are first defined. As used in the present disclosure, unless explicitly defined otherwise herein, each of the following terms shall have the meaning as set forth below.

The term "alkyl" refers to a saturated carbon chain having 1 to 20 carbon atoms, which can be linear, branched, or a combination thereof, unless the carbon chain is otherwise defined. Examples of alkyl includes methyl, ethyl, propyl, isopropyl, butyl, sec-butyl, tert-butyl, pentyl, hexyl, heptyl, octyl, etc.

Unless otherwise specifically stated in the description, the alkyl is optionally substituted. The term "unsaturated hydrocarbyl group" refers to a group containing at least one C=C double bond (alkenyl) or at least one C≡C triple bond (alkynyl). The "alkyl", "alkenyl" and "alkynyl" can be collectively referred to as "the hydrocarbyl group".

In the claims of the present disclosure, when "C1-C30 hydrocarbyl group" is described, it refers to the alkyl, alkenyl, or alkynyl having 1-30 carbon atoms (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 carbon atoms). "C₁-C₅ hydrocarbyl group ", "C₄-C₃₀ hydrocarbyl group ", "C₂-C₃₀ hydrocarbyl group " have analogous meanings.

When "alkyl" is described, it refers to a saturated hydrocarbyl having a specified number of carbon atoms (e.g., 2, 3, 4, 5, 6, 7, 8, 9, 10, 11, 12, 13, 14, 15, 16, 17, 18, 19, or 20 carbon atoms), which can be linear or branched, and is generally a chain group without a cyclic structure. The alkyl satisfying the aforementioned carbon atom number fall within the scope of this term.

When "alkenyl" is described, it refers to an alkenyl group having the specified number of carbon atoms (e.g., 2, 3, 4, 5, 6, 7, 8, 9, 10, 11, 12, 13, 14, 15, 16, 17, 18, 19, or 20 carbon atoms). The group can be linear or have a branched structure, and is generally a chain group without cyclic structures. All alkenyl groups satisfying the aforementioned carbon atom number fall within the scope of this term. In different embodiments of the present disclosure, the alkenyl can be derived from a monoalkene or a polyalkene (e.g., a diene).

When "C₂-C₃₀ linear or branched alkenyl" is described, it refers to the alkenyl having the specified number of carbon atoms (e.g., 2, 3, 4, 5, 6, 7, 8, 9, 10, 11, 12, 13, 14, 15, 16, 17, 18, 19, or 20 carbon atoms). The group can be linear or have a branched structure, and is generally a chain group without cyclic structures. All alkenyl satisfying the aforementioned carbon atom number fall within the scope of this term. In different embodiments of the present disclosure, the alkenyl can be monoalkenyl or polyalkenyl (e.g., diene).

When "C₁-C₁₀ alkylene" is described, it refers to the alkylene having 1 to 10 carbon atoms (e.g., 1, 2, 3, 4, 5, 6, 7, 8, 9, or 10 carbon atoms). The alkylene can be linear or branched, and is generally a chain group without a cyclic structure.

In the present application, when the definition of a divalent group includes "absent", it refers to the adjacent structural moieties linked by the divalent group are directly connected *via* a chemical bond.

### Ionizable lipid

As used herein, the terms "ionizable lipid of the present disclosure" and "ionizable cationic lipid of the present disclosure" are used interchangeably, and both refer to the lipid compounds having the structure of Formula I, or pharmaceutically acceptable salt, tautomer, or stereoisomer thereof.

The ionizable lipid becomes protonated and converted into a cationic lipid at a relatively low pH value, whereas it is converted back into a helper phospholipid under normal physiological pH conditions. The helper phospholipid has less interaction with the anionic cell membranes of blood cells, thereby enhancing the biocompatibility of the lipid nanoparticles. After the lipid nanoparticles are endocytosed by cells, the pH value in the endosomes is relatively low, causing the lipids to become protonated and positively charged. This impairs or even disrupts the stability of the membrane structure, thereby facilitating the endosomal escape of the lipid nanoparticles. In summary, the pH-sensitive property of the lipids is conducive to the *in vivo* delivery of lipid nanoparticles loaded with bioactive ingredients (e.g., mRNA molecules).

### Auxiliary lipid

As used herein, the term "auxiliary lipid" refers to a type of lipid other than the ionizable lipid in a lipid nanoparticle, including helper phospholipids, sterols, polymer-conjugated lipids, or a combination thereof. The auxiliary lipid is mainly used to improve the performance of lipid nanoparticles, such as stability, delivery efficiency, tolerability and biodistribution, etc.

In some embodiments, the helper phospholipid includes (but is not limited to)
1,2-distearoyl-sn-glycero-3-phosphocholine (DSPC),
1,2-dioleoyl-sn-glycero-3-phosphoethanolamine (DOPE), dioleoylphosphatidylcholine (DOPC), 1,2-dioleoyl-sn-glycero-3-phosphocholine,
1,2-dipalmitoyl-sn-glycero-3-phosphocholine,
1,2-dipalmitoyl-sn-glycero-3-phosphoethanolamine,
1,2-dimyristoyl-sn-glycero-3-phosphoethanolamine, sodium
1,2-dioleoyl-sn-glycero-3-phospho-rac-(1-glycerol), 1,2-dipalmitoylphosphatidylglycerol,
1-palmitoyl-2-oleoylphosphatidylcholine, 1-palmitoyl-2-oleoylphosphoethanolamine,
distearoyl phosphoethanolamine, 1-stearoyl-2-oleoylphosphatidylcholine,
1-stearoyl-2-oleoylphosphoethanolamine, or a combination thereof.

In a preferred embodiment of the present disclosure, the helper phospholipid is DSPC (1,2-distearoyl-sn-glycero-3-phosphocholine, also named distearoyl phosphatidylcholine). DSPC is a commonly used phosphatidylcholine; the tail group of DSPC is a saturated alkane chain, with a melting point of -54 °C and a cylindrical morphology. It forms a lamellar structure in lipid nanoparticles, thereby rendering the structure of the lipid nanoparticles more stable.

In a preferred embodiment of the present disclosure, the helper phospholipid is DOPE (1,2-dioleoyl-sn-glycero-3-phosphoethanolamine, also named dioleoyl phosphatidylethanolamine). DOPE is a commonly used phosphatidylethanolamine; the tail group of DOPE is two unsaturated alkane chains, with a melting point of -30°C and a conical morphology. It readily forms an inverted hexagonal phase in lipid nanoparticles, which destabilizes the endosomal membrane and facilitates the endosomal escape of the lipid nanoparticles.

In some embodiments, the sterol includes (but is not limited to) , cholesterol or cholesterol derivatives. Cholesterol can modulate the integrity and rigidity of the lipid membrane, thereby enhancing the stability of lipid nanoparticles. While the structural characteristics of cholesterol derivatives can affect the delivery efficiency and biodistribution of lipid nanoparticles, for example, the chain length of the hydrophobic tail group, the flexibility of the sterol ring, and the polarity of the hydroxyl group in cholesterol analogs. Cholesterol also affects the morphology of lipid nanoparticles, whereas cholesterol derivatives enable the formed lipid nanoparticles to adopt a lipid-separated multilamellar polyhedral structure rather than a spherical morphology. At same time, cholesterol also affects the targeting selectivity of lipid nanoparticles: lipid nanoparticles containing cholesteryl oleate exhibit higher selectivity for liver endothelial cells than for hepatocytes; whereas those containing cholesterol with oxidatively modified tail groups show higher accumulation in liver endothelial cells and Kupffer cells than in hepatocytes.

In some embodiments, the polymer-conjugated lipid is a polyethylene glycol (PEG)-conjugated lipid, also referred to as a pegylated lipid or PEGylated lipid. PEGylated lipids exert multiple effects on the properties of lipid nanoparticles: the dosage of PEGylated lipids affects the particle size and zeta potential of lipid nanoparticles, reduces particle aggregation to enhance the stability of lipid nanoparticles, decreases the rate of particle clearance mediated by the kidneys and the Mononuclear Phagocyte System (MPS), thereby prolonging the circulation time of the particles; the functional groups on the surface can be modified with ligands to improve targeted delivery capability. The molar mass and lipid chain length affect the properties of PEGylated lipids. Both DMG-PEG2000 and DSG-PEG2000 are neutral phospholipids with saturated alkyl chain lengths of C14, C16, or C18, respectively. However, DMG-PEG2000 can dissociate from lipid nanoparticles more rapidly, which facilitates cellular uptake of the nanoparticles and endosomal escape. Thus, DMG-PEG2000 exhibits superior delivery efficiency compared with DSG-PEG2000.

In a preferred embodiment of the present disclosure, the auxiliary lipid is a combination of DSPC, cholesterol, and DMG-PEG2000.

### Lipid nanoparticle (LNP)

As used herein, the terms "lipid nanoparticle", "lipid nanoparticulate" or "LNP" refer to particles with a diameter of about 5 to 500 nm. In some embodiments, the lipid nanoparticle contains one or more active agents (bioactive substances). In some embodiments, the lipid nanoparticle comprises nucleic acids. In some embodiments, the nucleic acids are condensed inside the nanoparticle with cationic lipids, polymers or multivalent small molecules, as well as an outer lipid coating that interacts with the biological environment. Due to the repulsion between phosphate groups, nucleic acids are inherently rigid polymers and tend to adopt an elongated conformation. In cells, to cope with volume constraints, DNA can package itself under appropriate solution conditions with the assistance of ions and other molecules. Generally, DNA condensation is defined as the collapse of extended DNA strands into compact, ordered particles containing only one or a few molecules. By binding to phosphate groups, cationic lipids can condense DNA by neutralizing phosphate charges and enabling its tight packing.

In some embodiments, the bioactive substance is encapsulated in the LNP. In some embodiments, the bioactive substance can be an anionic compound, including but not limited to DNA, RNA (messenger RNA, transfer RNA, ribosomal RNA, microRNA, etc.), natural and synthetic oligonucleotides (including antisense oligonucleotides, interfering RNA, and small interfering RNA), nucleoproteins, peptides, nucleic acids, ribozymes, DNA-containing nucleoproteins, e.g., fully or partially deproteinized viral particles (virions), and oligomeric and polymeric anionic compounds (e.g., acidic polysaccharides and glycoproteins) other than DNA. In some embodiments, the bioactive substance can be mixed with an adjuvant.

In LNP vaccine products, the bioactive substance is typically contained within the interior of the LNP. In some embodiments, the bioactive substance comprises nucleic acids. Generally, water-soluble nucleic acids are condensed with cationic lipids or polycationic polymers inside the particles, while the particle surface is enriched with helper phospholipids or PEG-lipid derivatives. Additional ionizable cationic lipids can also be located on the surface. Upon entering the lysosome of the cell, the ionizable cationic lipids become positively charged *via* ionization in the acidic environment of the lysosome, interact with the lysosomal membrane, and thereby facilitate endosomal escape.

With respect to LNPs, ionizable lipids can have different properties or functions. Owing to the pKa of amino groups, the ionizable lipid molecules will become protonated and positively charged when the external pH is lower than the pKa of the lipids. Under these conditions, the lipid molecules can electrostatically bind to the phosphate groups of nucleic acids, which enables LNP formation and nucleic acid encapsulation, while the LNPs exhibit a substantially neutral surface charge in biological fluids (e.g., blood) at physiological pH. A high surface charge of LNPs is associated with toxicity, rapid clearance by fixed and free macrophages in the circulation, hemolytic toxicity, and immune activation (Filion et al., Biochim Biophys Acta. 23 October 1997; 1329 (2): 345-56).

In some embodiments, the pKa can be sufficiently high to enable the ionizable cationic lipid to adopt a positively charged form at the acidic endosomal pH. In this way, the cationic lipid can bind to endogenous endosomal anionic lipids, facilitating the formation of membrane-lytic non-bilayer structures, e.g., the hexagonal HII phase, thereby achieving more efficient intracellular delivery. In some embodiments, the pKa ranges from 6.2-6.5. For example, the pKa can be about 6.2, about 6.3, about 6.4, or about 6.5. Unsaturated tails also contribute to the ability of the lipid to form non-bilayer structures (Jayaraman et al., Angew Chem Int Ed Engl, 20 August 2012; 51 (34): 8529-33).

The release of nucleic acids in the LNP formulation, as well as other characteristics such as liposome clearance rate and circulation half-life, can be modulated by the presence of polyethylene glycol and/or sterols (e.g., cholesterol) or other potential additives in the LNP, along with the overall chemical structure (including the pKa of any ionizable cationic lipids that are part of the formulation).

In one aspect of the present disclosure, provides a lipid nanoparticle (LNP), the lipid nanoparticle comprises the ionizable lipid, or pharmaceutically acceptable salt, tautomer or stereoisomer thereof according to the first aspect of the present disclosure. Further, the lipid nanoparticle also comprises one or more auxiliary lipids, the auxiliary lipids include helper phospholipids, steroids, and polymer-conjugated lipids.

### Lipid nanoparticle pharmaceutical formulation

In another aspect of the present disclosure, provides a lipid nanoparticle pharmaceutical formulation (or a lipid nanoparticle pharmaceutical composition, or an LNP composition). The lipid nanoparticle pharmaceutical formulation comprises the lipid nanoparticle according to the third aspect of the present disclosure, a bioactive substance encapsulated in the lipid nanoparticle, and a pharmaceutically acceptable carrier. The lipid nanoparticle pharmaceutical formulation is used for delivering a bioactive substance to the cells of a subject in need thereof.

In some embodiments, the bioactive substance is encapsulated in the LNP. In some embodiments, the bioactive substance can be an anionic compound, including but not limited to DNA, RNA (messenger RNA, transfer RNA, ribosomal RNA, microRNA, etc.), natural and synthetic oligonucleotides (including antisense oligonucleotides, interfering RNA and small interfering RNA), nucleoproteins, peptides, nucleic acids, ribozymes, DNA-containing nucleoproteins, e.g., complete or partially deproteinized viral particles (virions), as well as oligomeric and polymeric anionic compounds other than DNA (e.g., acidic polysaccharides and glycoproteins). In some embodiments, the bioactive substance can mix with an adjuvant.

In some embodiments, the LNP composition comprises: a nucleic acid; the ionizable cationic lipid having the structure represented by Formula (I); a helper phospholipid (e.g., DSPC, DOPE, DOPC, or a combination thereof); a sterol (e.g., cholesterol, a cholesterol derivative, or a phytosterol such as β-sitosterol); and a polymer-conjugated lipid (e.g., DMG-PEG2000). In some embodiments, the LNP composition comprises: a nucleic acid; the ionizable cationic lipid having the structure represented by Formula (I), in an amount of 30-65% of the total lipids in the composition (molar ratio, the same below); a helper phospholipid (e.g., DSPC, DOPE, DOPC, or a combination thereof), in an amount of 5-30% of the total lipids in the composition; a sterol (e.g., cholesterol, a cholesterol derivative, or a phytosterol such as β-sitosterol), in an amount of 30-55% of the total lipids in the composition; and a polymer-conjugated lipid (e.g., DMG-PEG2000), in an amount of 1-5% of the total lipids in the composition. And, in the LNP composition, the molar ratio of the ionizable N atoms in the ionizable lipid molecules to the phosphate groups in the nucleic acid molecules is (2~10):1, further preferably (4~8):1.

In a preferred embodiment of the present disclosure, the LNP composition comprises: a nucleic acid; the ionizable cationic lipid having the structure represented by Formula (I); a helper phospholipid (e.g., DSPC, DOPE, DOPC, etc., or a combination thereof); a sterol (e.g., cholesterol, a cholesterol derivative, or a phytosterol such as β-sitosterol); and a polymer-conjugated lipid (e.g., DMG-PEG2000, etc.). In a more preferred embodiment of the present disclosure, the LNP composition comprises: a nucleic acid; the ionizable cationic lipid having the structure represented by Formula (I); a helper phospholipid (e.g., DSPC, DOPE, DOPC, etc., or a combination thereof); a sterol (e.g., cholesterol, a cholesterol derivative, or a phytosterol such as β-sitosterol); and a polymer-conjugated lipid (e.g., DMG-PEG2000, etc).

As used herein, the terms "encapsulation" and "encapsulated" refer to the incorporation of mRNA, DNA, siRNA, or other nucleic acid drugs within or associated with lipid nanoparticles. As used herein, the term "encapsulation" refers to complete encapsulation or partial encapsulation. For example, mRNA can be selected to treat and/or prevent the associated diseases when a lipid nanoparticle composition comprising mRNA is administered to a subject in need thereof.

As used herein, the term "pharmaceutically acceptable carrier" includes, but is not limited to, any adjuvant, carrier, excipient, scintillant, sweetener, diluent, preservative, dye/colorant, flavor enhancer, surfactant, wetting agent, dispersant, suspending agent, stabilizer, isotonic agent, solvent, or emulsifier approved by the Food and Drug Administration for use in humans or livestock.

### Method for preparing a lipid nanoparticle pharmaceutical formulation

In another aspect of the present disclosure, provides a method for preparing a lipid nanoparticle pharmaceutical formulation. The method comprises: (a) mixing the ionizable lipid according to the first aspect of the present disclosure and an optional auxiliary lipid with an organic solvent to obtain a lipid organic phase; (b) mixing a bioactive substance with an aqueous solvent to obtain an aqueous phase containing the bioactive substance;(c) mixing the lipid organic phase in step (a) with the aqueous phase in step (b) to obtain the lipid nanoparticle pharmaceutical formulation. Further, the method also comprises step (d): purifying, concentrating and sterilizing by filtration the lipid nanoparticle pharmaceutical formulation obtained in step (c).

In some embodiments, the organic solvent includes (but is not limited to) ethanol, methanol, isopropanol, acetonitrile, dimethylformamide, dimethyl sulfoxide, dioxane, or tetrahydrofuran, or a combination thereof. In some embodiments, the lipid organic phase comprises a small percentage of water or a pH buffer solution. The lipid organic phase can contain up to 60% by volume of water, e.g., up to about 0.05%, 0.1%, 0.5%, 1%, 2%, 3%, 4%, 5%, 10%, 15%, 20%, 25%, 30%, 35%, 40%, 45%, 50%, 55%, or 60% by volume of water. In one embodiment, the lipid organic phase comprises water in an amount ranging from about 0.05% to 60% by volume, e.g., ranging from about 0.05% to 50%, from about 0.05% to 40%, or from about 5% to 20% by volume of water.

In some embodiments, the lipid organic phase comprises a single type of lipid, e.g., an ionizable cationic lipid, a helper phospholipid, a sterol, or a polymer-conjugated lipid. In some embodiments, the lipid organic phase comprises multiple types of lipids. In one embodiment of the present disclosure, the lipid organic phase comprises the ionizable cationic lipid having the structure represented by Formula (I), a helper phospholipid (e.g., DSPC, DOPE, DOPC, or a combination thereof), a sterol (e.g., cholesterol, a cholesterol derivative, or a phytosterol such as β-sitosterol), and a polymer-conjugated lipid (e.g., DMG-PEG2000). In a preferred embodiment of the present disclosure, the lipid organic phase comprises the ionizable cationic lipid having the structure represented by Formula (I), a helper phospholipid (e.g., DSPC, DOPE, DOPC, or a combination thereof), a sterol (e.g., cholesterol, a cholesterol derivative, or a phytosterol such as β-sitosterol), and a polymer-conjugated lipid (e.g., DMG-PEG2000). In a more preferred embodiment of the present disclosure, the lipid organic phase comprises the ionizable cationic lipid having the structure represented by Formula (I), a helper phospholipid (e.g., DSPC, DOPE, DOPC, or a combination thereof), a sterol (e.g., cholesterol, a cholesterol derivative, or a phytosterol such as β-sitosterol), and a polymer-conjugated lipid (e.g., DMG-PEG2000). In a specific embodiment of the present disclosure, the lipid organic phase comprises the ionizable cationic lipid having the structure represented by Formula (I), DSPC, cholesterol, and DMG-PEG2000.

In some embodiments, the aqueous solvent is water. In some embodiments, the aqueous solvent is an aqueous buffer solution with a pH ranging from 3 to 8 (e.g., a pH of about 3, about 4, about 5, or about 6, etc.). A bioactive substance, e.g., a nucleic acid (e.g., mRNA), is dissolved in the aqueous solvent to obtain an aqueous phase containing the bioactive substance. The aqueous phase can contain a small percentage of a water-miscible organic solvent. The aqueous phase can contain up to 60% by volume of at least one water-miscible organic solvent, e.g., up to about 0.05%, 0.1%, 0.5%, 1%, 2%, 3%, 4%, 5%, 6%, 7%, 8%, 9%, 10%, 15%, 20%, 25%, 30%, 35%, 40%, 45%, 50%, 55%, 60%, or any percentage by volume in between of an organic solvent (e.g., a water-miscible organic solvent). In one embodiment, the aqueous phase contains an organic solvent in an amount ranging from about 0.05% to 60% by volume, e.g., the organic solvent (e.g., a water-miscible organic solvent) in an amount ranging from about 0.05% to 50%, from about 0.05% to 40%, or from about 5% to 20% by volume. The aqueous buffer solution can be a citrate buffer, a Tris-HCl buffer solution, a sodium acetate buffer solution, a PBS buffer solution, or a combination thereof. In some embodiments, the aqueous buffer solution is a citrate buffer with a pH ranging from 4 to 6 (e.g., a pH of about 4, about 5, or about 6). In one embodiment, the aqueous buffer solution is a citrate buffer with a pH of about 4.

In some embodiments, the solution comprising the mixture of the lipid organic phase and the aqueous phase containing the bioactive substance, which contains the LNP suspension, can be diluted. In some embodiments, the pH of the solution comprising the mixture of the lipid organic phase and the aqueous phase containing the bioactive substance, which contains the LNP suspension, can be adjusted. The LNP suspension can be diluted or its pH can be adjusted by adding water, acid, base, or an aqueous buffer. In some embodiments, no dilution or pH adjustment of the LNP suspension is performed. In some embodiments, both dilution and pH adjustment are performed on the LNP suspension.

In some embodiments, excess reagents, solvents, and unencapsulated nucleic acids can be removed from the LNP suspension by tangential flow filtration (TFF) (e.g., diafiltration). Organic solvents (e.g., ethanol) and buffers can also be removed from the LNP suspension by TFF. In some embodiments, the LNP suspension is subjected to dialysis. In some embodiments, the LNP suspension is subjected to TFF. In some embodiments, the LNP suspension is subjected to both dialysis and TFF.

### The main advantages of the present disclosure include:

(a) The ionizable lipids in the present disclosure can form stable nanoparticles with other components, e.g., helper phospholipids (DSPC, DOPE, DOPC, DOPS, etc.), sterols (e.g., cholesterol or cholesterol derivatives), and PEG derivatives (e.g., DMG-PEG lipids, or DMG-PEG substances modified with other groups, or other PEG derivatives).
(b) After the ionizable lipids in the present disclosure form nanoparticles with other components, the nanoparticles can encapsulate mRNA with uniform particle size, high encapsulation efficiency and good stability. They can improve the transfection efficiency of mRNA in targeted tissues or cells with low toxicity, thus making the preventive and therapeutic effects of mRNA vaccines/medicines more prominent.

The present disclosure was further illustrated below with reference to specific examples. It should be understood that these examples are merely intended to illustrate the present disclosure and not to limit the scope thereof. For the experimental methods without specified conditions in the following examples, they were generally carried out in accordance with conventional conditions or the conditions recommended by the manufacturers. Unless otherwise specified, all percentages and parts were by weight.

### Example 1

### The preparation of ionizable lipid

### 1.1: Synthesis and characterization of compound AXT-8:

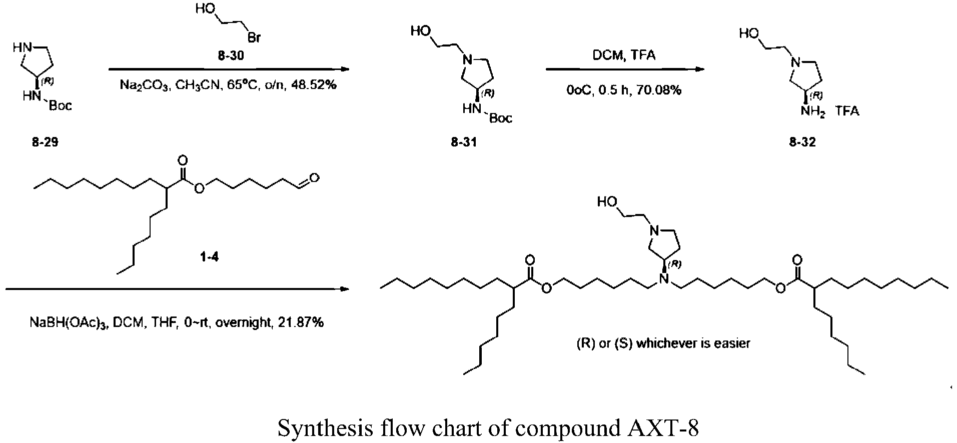

### 1.1.1: Synthesis and characterization of Product 8-31

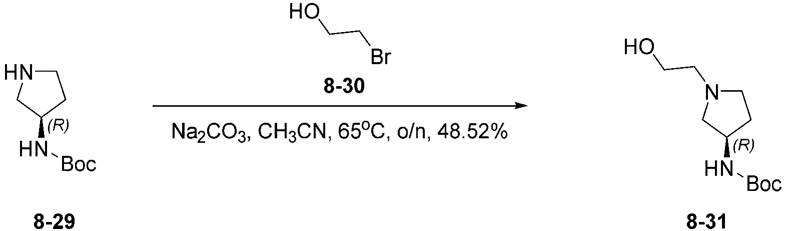

Tert-butyl N-[(3R)-pyrrolidin-3-yl]carbamate (1.2 g, 6.443 mmol, 1 equiv), 2-bromoethanol (1.21 g, 9.665 mmol, 1.5 equiv), Na₂CO₃ (1.37 g, 12.886 mmol, 2 equiv) and CH₃CN (24 mL) were placed in a 40 mL round-bottom flask. Under the protection of inert nitrogen atmosphere, the resulting solution was stirred overnight at 65 °C.

Then, the reaction mixture was cooled to ambient temperature and filtered, and the filtrate was concentrated under reduced pressure. The residue was solubilized with 50 mL of water, and the resulting solution was extracted with 3 × 50 mL of ethyl acetate. The organic layers were combined, dried over anhydrous sodium sulfate, and concentrated.

As a result, tert-butyl N-[(3R)-1-(2-hydroxyethyl)pyrrolidin-3-yl]carbamate (0.72 g, yield 48.52%) was obtained as a colorless oil. The mass spectrometry and nuclear magnetic resonance data were shown as follows:
LC-MS-8-31: (ES, m/z): 231 [M+H]+;
H-NMR-8-31: 1H NMR (300 MHz, Chloroform-d, ppm) δ 4.998 (brs, 1H), 4.249 (brs, 1H), 3.702 (t, J = 5.4 Hz, 2H), 3.025 (brs, 1H), 2.776-2.725 (m, 4H), 2.495-2.381 (m, 2H), 2.353-2.294 (m, 1H), 1.688 (t, J = 8.4 Hz, 1H), 1.464 (s, 9H).

### 1.1.2: Synthesis and characterization of Product 8-32

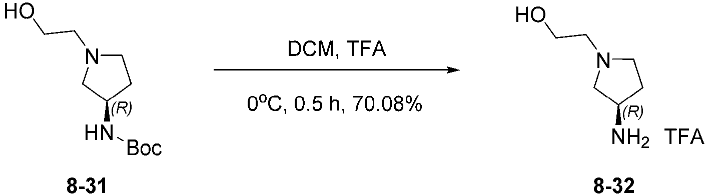

Tert-butyl N-[(3R)-1-(2-hydroxyethyl)pyrrolidin-3-yl]carbamate (0.72 g, 3.126 mmol, 1 equiv) and DCM (7.2 mL) were placed in a 100 mL three-necked round-bottom flask, which was protected by an inert nitrogen atmosphere.

Then, at 0 °C, trifluoroacetaldehyde (1.45 mL) was added dropwise. The resulting solution was stirred at 0 °C for 30 minutes and monitored by LCMS. The resulting mixture was concentrated.

As a result, 2-[(3R)-3-aminopyrrolidin-1-yl]ethanol; trifluoroacetaldehyde adduct (0.5 g, yield: 70.08%) was obtained as a pale yellow oil. The results of mass spectrometry and nuclear magnetic resonance characterization were shown as follows:
LC-MS-8-32: (ES, m/z): 131 [M+H]+;
H-NMR-8-32: 1H NMR (300 MHz, Chloroform-d, ppm) δ 4.128 (brs, 1H), 3.830 (t, J = 4.8 Hz, 2H), 3.653-3.392 (m, 4H), 3.353-3.279 (m, 2H), 2.612-2.488 (m, 1H), 2.235-2.142 (m, 1H).

### 1.1.3: Synthesis and characterization of final product

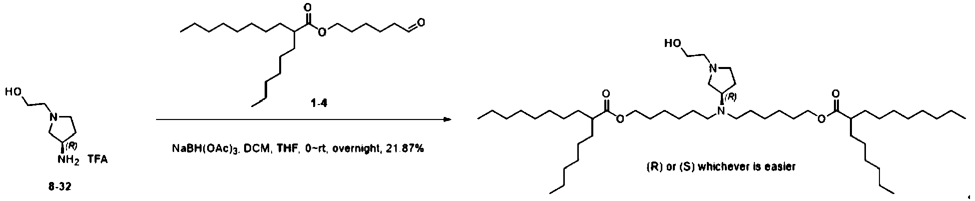

Synthesis procedures:
1) 2-[(3R)-3-aminopyrrolidin-1-yl]ethanol was placed in a 500 mL three-necked round-bottom flask. Under the protection of an inert nitrogen atmosphere, trifluoroacetaldehyde (300 mg, 1.315 mmol, 1 equiv), 6-oxohexyl-2-hexyldecanoate (1025.43 mg, 2.893 mmol, 2.2 equiv), DCM (15 mL) and THF (15 mL) were successively added. The resulting solution was stirred at room temperature for 0.5 hours;
2) At 0°C, diacetyl peroxide was added dropwise; sodium acetate borate (835.82 mg, 3.945 mmol, 3 equiv) was added with the dropping duration controlled within 5 minutes;
3) Stirring was continued at 0°C for 0.5 hours, followed by stirring at room temperature overnight;
4) 50 mL of saturated NH₄Cl solution was added to quench the reaction;
5) The resulting mixture was extracted with 3×50 mL of DCM, and the organic layers were combined.
6) The combined organic phase was dried over anhydrous sodium sulfate and then concentrated under reduced pressure.
7) The residue was further purified by flash preparative HPLC under the following conditions (IntelFlash-1): column, C18 silica gel; mobile phase: A: H₂O (0.05%TFA) B:CH₃CN= 55, linearly adjusted to H₂O (0.05%TFA) B:CH₃CN=95 within 15 minutes and maintained for 10 min; detector, UV detector at 220 nm; and
8) The qualified fractions were combined and concentrated. The residue was dissolved in 50 mL of DCM, and the organic phase was washed with 2 × 50 mL of NaHCO₃ (aq, 2M), 1 × 50 mL of water and 50 mL of brine, then dried over anhydrous Na₂SO₄.

The filtrate obtained after filtration was concentrated under reduced pressure. Thus, 6-({6-[(2-hexyldecyl)oxy]hexyl}[(3R)-1-(2-hydroxyethyl)pyrrolidin-3-yl]amino)hexyl 2-hexyldecanoate (0.2321 g, yield: 21.87%) was obtained as a yellow oil. The characterization results of mass spectrometry, nuclear magnetic resonance and HPLC were shown as follows.
LC-MS: (ES, m/z): 807.8 [M+H]⁺;
H-NMR: 1H NMR (300 MHz, Chloroform-d, ppm) δ 4.062 (t, J = 6.6 Hz, 4H), 3.639 (t, J = 5.1 Hz, 2H), 3.419 (t, J = 6.9 Hz, 1H), 2.745-2.569 (m, 6H), 2.473 (t, J = 7.8 Hz, 4H), 2.337-2.279 (m, 2H), 2.062-1.926 (m, 1H), 1.822-1.708 (m, 1H), 1.692-1.504 (m, 8H), 1.492-1.188 (m, 56H), 0.876 (t, J = 5.7 Hz, 12H);

The results of purity analysis of AXT-8 by HPLC-CAD showed that the retention time of the product was 18.039 min, with a purity of over 94%.

### Example 2

### LNP-mRNA was assembled by encapsulating mRNA with ionizable lipids

In the present disclosure, LNP-mRNA was prepared by encapsulating mRNA with ionizable lipids, and the steps included:
1) Ionizable lipid (AXT-8), DSPC (distearoylphosphatidylcholine, 1,2-distearoyl-sn-glycero-3-phosphocholine), cholesterol, and DMG-PEG2000 were mixed at different ratios in an ethanol phase and were fully homogenized to serve as the organic phase;
2) mRNA was dissolved in a sodium citrate solution (pH=4.0) and was fully homogenized to serve as the aqueous phase;
3) The aqueous phase and the organic phase were separately transferred into suitable BD syringes, with air bubbles exhausted as thoroughly as possible;
4) Using a PNI nanoparticle preparation instrument, mixing was performed under the conditions of an aqueous-to-organic phase volume ratio of 3:1 and a flow rate of 12 mL/min. Purification, concentration, and sterile filtration were sequentially carried out to obtain the mRNA-encapsulated lipid nanoparticle (LNP-mRNA) product; and
5) Physicochemical quality control was conducted on the final LNP-mRNA product.

Physicochemical quality control methods and results for LNP-mRNA:
1) Particle size and PDI (Polydispersity Index): The particle size and polydispersity of LNPs were determined using a nanoparticle size analyzer, with the results detailed in Figure 1 and Figure 2.
2) Encapsulation Efficiency (EE%): Total mRNA and free mRNA were separately stained with RiboGreen, followed by detection using a microplate reader. The encapsulation efficiency was calculated accordingly, with the results detailed in Figure 3.
3) pH: The pH value of the final product was measured using a pH meter, and the result was 7.2-7.4.
4) Osmotic Pressure: The osmotic pressure of LNP-mRNA was determined using a freezing point osmometer. At a detection concentration of 100 µg/mL, the osmotic pressure was 280-310 mOsmol/kg.
5) mRNA Integrity: The integrity (i.e., purity of mRNA) of the encapsulated mRNA was tested using Agilent Fragment Analyzers. The purity was determined to be greater than 90% with an RNA kit (15 nt).

### Example 3

### In Vitro Expression Assay of LNP-mRNA

The present disclosure also conducted cell expression screening experiments for LNP-mRNA. The specific steps were as follows:
1) Cell Plating: 293T cells were digested, and the cell density was adjusted to 2×10⁴/well, then seeded into a 96-well plate at a volume of 100 µL/well. The plate was then incubated overnight in a cell culture incubator.
2) Experimental Grouping: LNP (AXT-8)-Luc series products were used as the sample group; Lipofectamine Messenger MAX was set as the positive control group; and a mixture containing only 100 µL of medium and cells was used as the negative control group.
3) Cell Transfection: LNP-Luc did not require transfection. Taking 100 ng of mRNA per well as the maximum concentration, three-fold serial dilutions were performed to obtain six gradients in total, with three replicate wells set up for each sample. 10 µL of LNP-Luciferase at different dilution ratios was uniformly added to the 96-well cell plate, and the plate was incubated in the incubator after thorough mixing.
4) Kit Detection: After 48 h of incubation, the assay was performed in accordance with the protocol of the ONE-Glo^{™} EX Luciferase Assay System kit. The luciferase luminescence intensity was detected using a BioTek SYNERGY microplate reader, and the OD values were averaged for comparison.

The results were as shown in Figure 4. LNP (AXT-8)-Luc samples with different formulations were all capable of normal expression in cells, and the expression level increased with the rise in mRNA concentration. It can thus be concluded that the LNP-mRNA prepared from AXT-8 achieved high-efficiency expression *in vitro.*

### Example 4

### In Vitro Toxicity Assay of LNP-mRNA

Cytotoxicity assay, the specific steps were as follows:
1) Cell Plating: 293T cells were digested, and the cell density was adjusted to 2×10⁴ /well, then seeded into a 96-well plate at 100 µL per well. The plate was then incubated overnight in a cell culture incubator.
2) Experimental Grouping: LNP (AXT-8)-Luc was used as the sample group; 100 µL of medium alone was used as the blank control group; a mixture of 100 µL of medium and cells was used as the negative control group; and a mixture of 100 µL of medium and cells supplemented with the apoptosis inducer Staurosporine (STS) was used as the positive control group.
3) Cell Transfection: LNP-Luc did not require transfection. Using 100 ng of mRNA per well as the maximum concentration, three-fold serial dilutions were performed with buffer to obtain four gradients in total. 10 µL of LNP-Luc at each dilution was uniformly added to the 96-well cell plate, with three replicate wells set up for each sample, and the plate was incubated in the incubator after thorough mixing. 10 µL of 100 µM STS (diluted in PBS) was added to the 96-well cell plate, with six replicate wells set up. The plate was incubated in the incubator after thorough mixing.
4) Kit Detection: After 48 h of incubation, the assay was performed in accordance with the protocol provided by the CellTiter-Glo^{®} Luminescent Cell Viability Assay kit. The luminescence intensity was detected using a BioTek SYNERGY microplate reader, and the OD values were averaged for comparison. The cell inhibition rate was calculated using the formula: (Negative Control Group - Sample Group/Positive Control Group) ÷ (Negative Control Group -Blank control group ) × 100%, and the dose-response-inhibition curve was plotted.

The results were as shown in Figure 5. For the series of LNP-mRNA products formulated with AXT-8, no significant effect on cell proliferation was observed when the concentration was less than 1 µg/mL, indicating that there was no obvious cytotoxicity within the tested concentration range.

### Example 5

### In Vivo Expression Assay of LNP-mRNA

To illustrate the *in vivo* expression capacity and toxicity of LNP-mRNA formulated with AXT-8, hEPO ELISA expression assays and toxicity experiments were further designed. The expression level and toxicity were determined in mice with intact innate immunity. The specific steps were shown as follows:
1) Encapsulation of LNP-mRNA: hEPO mRNA was dissolved in an aqueous buffer and mixed thoroughly to serve as the aqueous phase. Ionizable lipid (AXT-8), DSPC, cholesterol, and DMG-PEG2000 were separately dissolved in anhydrous ethanol, and mixed thoroughly at a specific ratio to serve as the organic phase. The aqueous phase and the organic phase were separately transferred into syringes. hEPO-LNP was prepared using a PNI microfluidic nanoparticle preparation instrument (the volume ratio of aqueous phase to organic phase was 3:1, and the flow rate was 12 mL/min). After concentration, purification, sterile filtration, and passing quality control, the LNP-hEPO was administered to mice *via* injection.
2) Mouse Tail Vein Injection: Mice were immobilized, and the tail vein was selected for injection. The injection dose of LNP-hEPO was 5 µg. LNP (AXT-8)-hEPO was designated as the sample group, while mice injected with solvent alone served as the negative control group. Two mice were injected per LNP-hEPO sample.
3) Submandibular Blood Collection in Mice: At 6 h and 24 h post-injection, submandibular blood collection was performed. The collected blood was placed into EDTA anticoagulant tubes and mixed gently, then labeled for subsequent use.
4) Serum Extraction: After whole blood collection, centrifugation was carried out at 2000 × g for 10 minutes. The supernatant was collected as plasma, aliquoted, and stored at -80 °C.
5) Determination of hEPO Expression Level Using Human Erythropoietin ELISA Kit: The assay was performed in accordance with the kit instructions, with two replicate wells set up for each serum sample. The OD values were measured using a microplate reader, and the expression levels were calculated based on the standard curve and dilution ratio. The conversion factor between IU and µg for the kit used was exemplified as follows: 1650IU = 11 µg.

The physicochemical property values of the LNP-hEPO formulation prepared with AXT-8, which can achieve successful expression in mice, as well as the *in vivo* hEPO expression concentrations were shown in Table 2.

**Table 2**

| Formulation | Particle Size (nm) | PDI | Encapsulation Efficiency (%) | Expression Level at 6 h (µg/mL) |
|---|---|---|---|---|
| LNP(AXT-8)-hEPO-1 | 95.19 | 0.091 | 94.81 | C |
| LNP(AXT-8)-hEPO-2 | 87.64 | 0.074 | 96.75 | C |

| | | | | |
|---|---|---|---|---|
| C: ≥0.1 and <1; D: <0.1 | | | | |

The results were as shown in Figure 6. The series of LNP (AXT-8)-hEPO products formulated with AXT-8 achieved successful expression in mice. This experiment demonstrated that the LNPs formed with AXT-8 could efficiently mediate the *in vivo* expression of mRNA.

### Example 6

### Expression Assay of LNP-mRNA in T Cell Lines

To illustrate the capacity of LNP-mRNA formulated with AXT-8 to transfect human T cell lines *in vitro,* experiments were designed to transfect JM cells and Jurkat cells with LNP-eGFP. Meanwhile, Lipofectamine^{™} MessengerMAX^{™} (Lipofectamine Messenger MAX, purchased from Thermo Fisher Scientific, product code: LMRNA001) was used as the positive control group to determine the eGFP expression level in the cells. The specific steps were as shown as follows:
1) Cell Plating: Cells were counted using the AO/PI method. The culture medium contained only FBS without antibiotics. JM cells or Jurkat cells were adjusted to a density of 1*10⁶/mL and seeded into a 24-well plate at 200 uL per well, corresponding to 2*10⁵/ well.
2) Cell Transfection: mRNA encapsulated with AXT-8 was added at a dose of 6 ug/well, and the medium was supplemented to 1 mL. The medium contained only FBS without adding antibiotics.
3) Flow Cytometry Analysis: After incubation at 37 °C for 24 h, flow cytometry analysis was performed. The cells were harvested separately into 1.5 mL EP tubes, labeled with sample names, centrifuged at 400 g for 5 min, and the supernatant was discarded. The cells were washed once with 1 mL of 2% FBS (prepared in PBS), centrifuged at 400 g for 5 min, and the supernatant was discarded. The corresponding antibody was added to the cell suspension and incubated for 20 min for staining. The cells were washed again with 1 mL of 2% FBS (prepared in PBS), centrifuged at 400 g for 5 min, and the supernatant was discarded. The cells were resuspended in 50 uL of 2% FBS (prepared in PBS) and subjected to flow cytometry analysis.

The results were as shown in Figure 7. The LNP-eGFP formulated with AXT-8 achieved successful expression in both JM cells and Jurkat cells.

### Example 7

### Expression Assay of LNP-mRNA in Human Primary T Cells

To illustrate the capacity of LNP-mRNA formulated with AXT-8 to transfect human T cell lines *in vitro,* experiments were designed to transfect human primary T cells with LNP-mCherry. Meanwhile, the transfection reagent Lipofectamine^{™} MessengerMAX^{™} (Lipofectamine Messenger MAX) was used as the positive control group to determine the mCherry expression level in the cells. The specific steps were shown below:
1) Isolation of Primary T Cells from Human PBMCs: Magnetic bead labeling was performed (the following procedure is exemplified with 10⁷ cells; the amount of corresponding reagents should be scaled up proportionally according to the total cell number). Every 10⁷ total cells were resuspended in 80 µL of buffer, followed by the addition of 20 µL of CD3 MicroBeads. The cell suspension was incubated for 15 minutes (2 to 8 °C) in a refrigerator, then washed with buffer. Up to 10⁸ cells were resuspended in 500 µL of buffer. The MS column was placed on a magnetic separator, and T cells were sorted using the MS column.
2) Transfection of Primary T Cells with AXT-8: The T cells were adjusted to a density of 1*10⁶/mL and seeded into a 24-well plate at 200 µL per well, corresponding to 2*10⁵/per well. AXT-8 was added at a concentration of 6 µg/mL, and the medium was supplemented with X-vivo medium to 1 mL. IL-2 was added to each well to a final concentration of 100 IU/mL. The cells were incubated at 37 °C for 24 hours prior to flow cytometry analysis.
3) Flow Cytometry Analysis: The cells were harvested separately into 1.5 mL EP tubes and labeled with sample names. The cell suspension was centrifuged at 400 g for 5 min, and the supernatant was discarded. The cells were washed once with 1 mL of 2% FBS (prepared in PBS), centrifuged again at 400 g for 5 min, and the supernatant was discarded. The corresponding antibody was added to the cell suspension and incubated for 20 min for staining. The cells were washed again with 1 mL of 2% FBS (prepared in PBS), centrifuged at 400 g for 5 min, and the supernatant was discarded. The cells were resuspended in 50 µL of 2% FBS (prepared in PBS) and subjected to flow cytometry analysis.

The results were as shown in Figure 8. The LNP-mCherry formulated with AXT-8 achieved successful expression in human primary T cells.

All documents mentioned in the present disclosure are incorporated herein by reference, as if each individual document were specifically and individually indicated to be incorporated by reference. In addition, it should be understood that, after reading the above teachings of the present disclosure, those skilled in the art can make various alterations or modifications to the present disclosure, and such equivalent forms shall also fall within the scope defined by the appended claims of the present disclosure.

## Claims

1. An ionizable lipid, or a pharmaceutically acceptable salt, a tautomer or a stereoisomer thereof, wherein, the ionizable lipid has the structure of Formula I below: 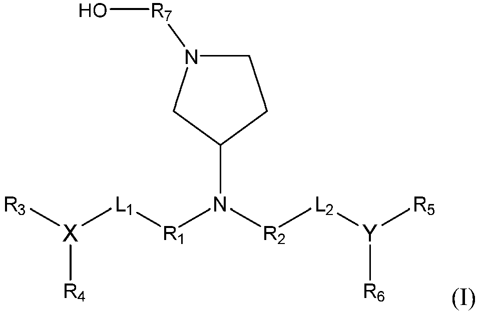 wherein,
R₁ and R₂ are each independently selected from -(CH₂)ₙ-, wherein n is a positive integer ranging from 1 to 14;
X and Y are each independently-CH- or N;
Li has the structure of -(L₁ₐ-L_{1b})- from right to left, or is absent, wherein, L₁ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-; L_{1b} is -(CH₂)ₙ-, wherein n is selected from 0, 1, 2, 3 or 4;
L₂ has the structure of -(L₂ₐ-L_{2b})- from left to right, or is absent; wherein, L₂ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-; L_{2b} is -(CH₂)ₙ-, wherein n is selected from 0, 1, 2, 3 or 4;
R₃, R₄, R₅ and R₆ are each independently H, CH₃, C₂-C₃₀ hydrocarbyl group, or -(CH₂)ₛ-R^{a}-(CH₂)_{g}-R^{b}-(CH₂)ₘ-CH₃, wherein, s, g are each independently selected from a positive integer ranging from 1 to 20, m is selected from a integer ranging from 0 to 20;
R^{a}, R^{b} are each independently absent or selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-;
and, R₃ and R₄ are not both H; and R₅ and R₆ are not both H;
R₇ is selected from C₁-C₅ hydrocarbyl group, or -(CH₂)ₘO(CH₂)ₙ-, wherein m, n are each independently selected from 1, 2, 3.

2. The ionizable lipid, or pharmaceutically acceptable salt, tautomer or stereoisomer thereof according to claim 1, wherein, the ionizable lipid has the structure represented by Formula (I-1) below: 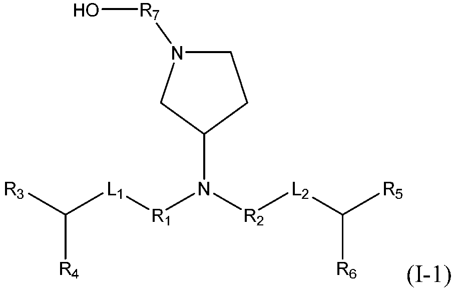 wherein,
R₁ and R₂ are each independently selected from -(CH₂)ₙ-, wherein n is a positive integer ranging from 1 to 14;
Li has the structure of -(L₁ₐ-L_{1b})- from right to left, or is absent, wherein, L₁ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-; L_{1b} is -(CH₂)ₙ-, wherein n is selected from 0, 1, 2, 3 or 4;
L₂ has the structure of -(L₂ₐ-L_{2b})- from left to right, or is absent; wherein, L₂ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-; L_{2b} is -(CH₂)ₙ-, wherein n is selected from 0, 1, 2, 3 or 4;
R₃, R₄, R₅ and R₆ are each independently C₂-C₂₀ hydrocarbyl group;
R₇ is selected from C₁-C₅ hydrocarbyl group, or -(CH₂)ₘO(CH₂)ₙ-, wherein m, n are each independently selected from 1, 2, 3.

3. The ionizable lipid, or pharmaceutically acceptable salt, tautomer or stereoisomer thereof according to claim 1, wherein, the ionizable lipid has the structure represented by Formula (I-2) below: 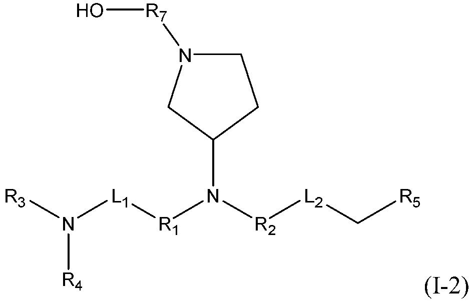 wherein,
R₁ and R₂ are each independently selected from -(CH₂)ₙ-, wherein n is a positive integer ranging from 1 to 14;
Li has the structure of -(L₁ₐ-L_{1b})- from right to left, or is absent, wherein, L₁ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-; L_{1b} is -(CH₂)ₙ-, wherein n is selected from 0, 1, 2, 3 or 4;
L₂ has the structure of -(L₂ₐ-L_{2b})- from left to right, or is absent; wherein, L₂ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-; L_{2b} is -(CH₂)ₙ-, wherein n is selected from 0, 1, 2, 3 or 4;
R₃ has the structure of -R₃ₐ-R_{3b}-R_{3c}-R_{3d}-R_{3c}, R₄ has the structure of -R₄ₐ-R_{4b}-R_{4c}-R_{4d}-R_{4c}, R₅ has the structure of -R₅ₐ-R₅₆-R_{5c}-R_{5d}-R_{5c};
wherein, R₃ₐ, R_{3c}, R₄ₐ, R_{4c}, R₅ₐ, R_{5c} are each independently -(CH₂)ₙ-, wherein n is selected from a positive integer ranging from 1 to 14;
R_{3b}, R_{3d}, R_{4b}, R_{4d}, R_{5b}, R_{5d} are each independently absent or selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, -(C=C)-(CH₂)-(C=C)-, -(C=C)-, -CH₂-, -(C≡C)-;
R_{3c}, R_{4c}, R_{5c} are each independently C₂-C₂₀ hydrocarbyl group;
R₇ is selected from C₁-C₅ hydrocarbyl group, or -(CH₂)ₘO(CH₂)ₙ-, wherein m, n are each independently selected from 1, 2, 3.

4. The ionizable lipid, or pharmaceutically acceptable salt, tautomer or stereoisomer thereof according to claim 1, wherein, the ionizable lipid has the structure represented by Formula (I-3) below: 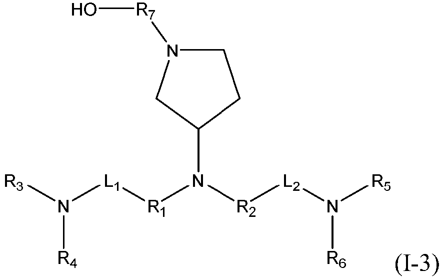 wherein,
R₁ and R₂ are each independently selected from -(CH₂)ₙ-, wherein n is a positive integer ranging from 1 to 14;
Li has the structure of -(L₁ₐ-L_{1b})- from right to left, or is absent, wherein, L₁ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-; L_{1b} is -(CH₂)ₙ-, wherein n is selected from 0, 1, 2, 3 or 4;
L₂ has the structure of -(L₂ₐ-L_{2b})- from left to right, or is absent; wherein, L₂ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-; L_{2b} is -(CH₂)ₙ-, wherein n is selected from 0, 1, 2, 3 or 4;
R₃ has the structure of -R₃ₐ-R_{3b}-R_{3c}-R_{3d}-R_{3c}, R₄ has the structure of -R₄ₐ-R_{4b}-R_{4c}-R_{4d}-R_{4c}; R₅ has the structure of -R₅ₐ-R₅₆-R_{5c}-R_{5d}-R_{5c}, R₆ has the structure of -R₆ₐ-R_{6b}-R_{6c}-R_{6d}-R_{6c};
wherein, R₃ₐ, R_{3c}, R₄ₐ, R_{4c}, R₅ₐ, R_{5c}, R₆ₐ and R_{6c} are each independently -(CH₂)ₙ-, wherein n is selected from a positive integer ranging from 1 to 14;
R_{3b}, R_{3d}, R_{4b}, R_{4d}, R_{5b}, R₅ₐ, R_{6b} and R_{6d} are each independently absent or selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, -(C=C)-(CH₂)-(C=C)-, -(C=C)-, -CH₂-, -(C≡C)-;
R_{3c}, R_{4c}, R_{5c}, R_{6c} are each independently C₂-C₂₀ hydrocarbyl group;
R₇ is selected from C₁-C₅ hydrocarbyl group, or -(CH₂)ₘO(CH₂)ₙ-, wherein m, n are each independently selected from 1, 2, 3.

5. The ionizable lipid, or pharmaceutically acceptable salt, tautomer or stereoisomer thereof according to claim 1, wherein, R₃, R₄, R₅ and R₆ are each independently C₅-C₁₅ alkyl.

6. The ionizable lipid, or pharmaceutically acceptable salt, tautomer or stereoisomer thereof according to claim 1, wherein, the ionizable lipid has the structure represented by Formula (I-1) below: 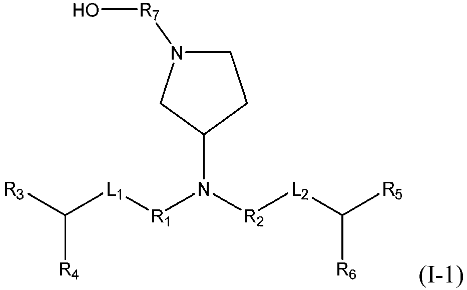 wherein,
R₁ and R₂ are each independently selected from -(CH₂)ₙ-, wherein n is a positive integer ranging from 4 to 8;
Li is selected from the following functional groups: -O-, -(C=O)O-, -O(C=O)-, -CH(OH)-;
L₂ is selected from the following functional groups: -O-, -(C=O)O-, -O(C=O)-, -CH(OH)-;
R₃ has the structure of -R₃ₐ-R_{3b}-R_{3c}-R_{3d}-R₃ₑ, R₄ has the structure of -R₄ₐ-R_{4b}-R_{4c}-R_{4d}-R₄ₑ; R₅ has the structure of -R₅ₐ-R_{5b}-R_{5c}-R_{5d}-R₅ₑ, R₆ has the structure of -R₆ₐ-R_{6b}-R_{6c}-R_{6d}-R₆ₑ;
wherein, R₃ₐ, R_{3c}, R₄ₐ, R_{4c}, R₅ₐ, R_{5c}, R₆ₐ and R_{6c} are each independently -(CH₂)ₙ-, wherein n is selected from a positive integer ranging from 1 to 14; and R₃, R₄, R₅ and R₆ each independently has 4-20 CH₂ structural moieties;
R_{3b}, R_{3d}, R_{4b}, R_{4d}, R_{5b}, R_{5d}, R_{6b} and R_{6d} are each independently absent or selected from the following functional groups: -(C=O)O-, -O(C=O)-, -(S-S)-, -(C=C)-(CH₂)-(C=C)-, -CH(OH)-;
R₃ₑ, R₄ₑ, R₅ₑ, R₆ₑ are each independently C₂-C₂₀ hydrocarbyl group;
R₇ is C₁-C₃ hydrocarbyl group, or -(CH₂)₂-O-(CH₂)₂-.

7. The ionizable lipid, or pharmaceutically acceptable salt, tautomer or stereoisomer thereof according to claim 1, wherein, the ionizable lipid has the structure represented by the Formula below:

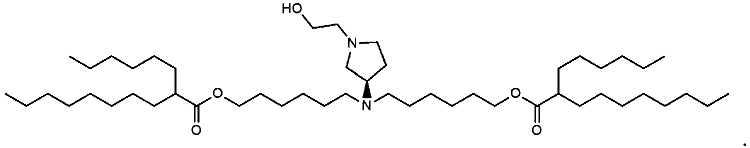

8. A method for preparing the ionizable lipid, or pharmaceutically acceptable salt, tautomer or stereoisomer thereof according to claim 1, wherein, the method comprises: method I, method II and method III;
wherein, method I comprises the following steps:
(S1) under the protection of an inert gas, compounds A1 and A2 are reacted to obtain compound A3;
(S2) under the protection of an inert gas, the tert-butoxycarbonyl (Boc) group of A3 is removed to obtain A4;
(S3) under the protection of an inert gas, A4 is reacted with A5 or A6 to obtain A7 (i.e., the compound represented by (I-1)); 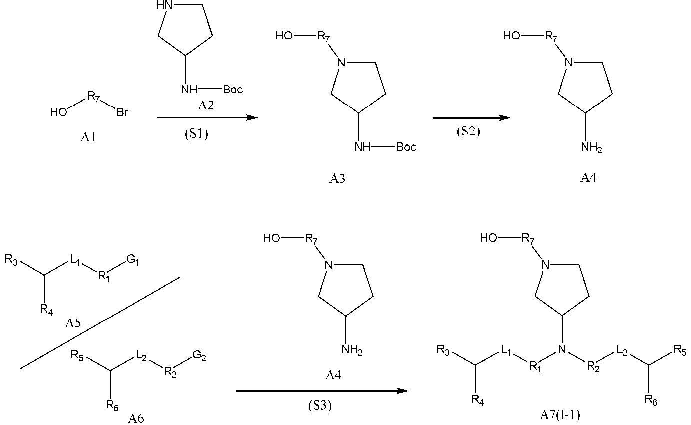
wherein, R₁ and R₂ are each independently selected from -(CH₂)ₙ-, wherein n is a positive integer ranging from 1 to 14;
G₁ and G₂ are each independently selected from an active functional group;
Li has the structure of -(L₁ₐ-L_{1b})- from right to left, or is absent, wherein, L₁ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-; L_{1b} is -(CH₂)ₙ-, wherein n is selected from 0, 1, 2, 3 or 4;
L₂ has the structure of -(L₂ₐ-L_{2b})- from left to right, or is absent; wherein, L₂ₐ is selected from the following functional groups: O, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-; L_{2b} is -(CH₂)ₙ-, wherein n is selected from 0, 1, 2, 3 or 4;
R₃, R₄, R₅ and R₆ are each independently C₂-C₂₀ hydrocarbyl group;
R₇ is selected from C₁-C₅ hydrocarbyl group, or -(CH₂)ₘO(CH₂)ₙ-, wherein m, n are each independently selected from 1, 2, 3;
method II comprises the following steps:
(D1) under the protection of an inert gas, compound B1 is reacted with B2 to obtain compound B3;
(D2) under the protection of an inert gas, compound B3 is reacted with B4 to obtain compound B5;
(D3) under the protection of an inert gas, compound B5 is reacted with B6 to obtain compound B7, i.e., the compound represented by (1-2); 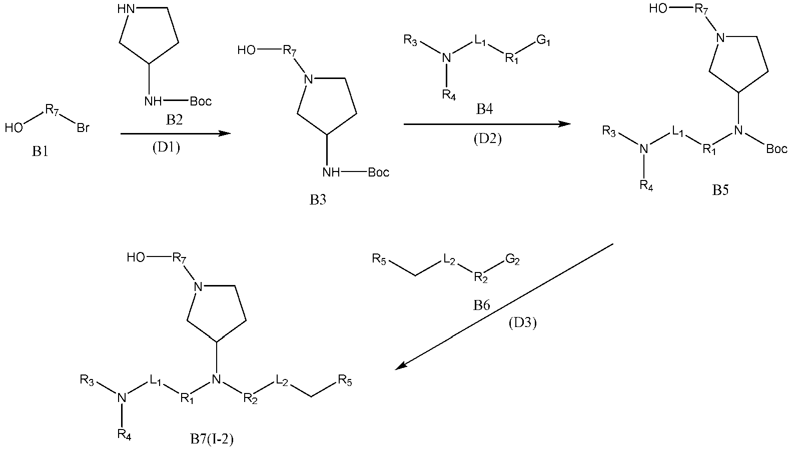
wherein, R₁ and R₂ are each independently selected from -(CH₂)ₙ-, wherein n is a positive integer ranging from 1 to 14;
G₁ and G₂ are each independently selected from an active functional group;
L₁ has the structure of -(L₁ₐ-L_{1b})- from right to left, or is absent, wherein, L₁ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-; L_{1b} is -(CH₂)ₙ-, wherein n is selected from 0, 1, 2, 3 or 4;
L₂ has the structure of -(L₂ₐ-L_{2b})- from left to right, or is absent; wherein, L₂ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-; L₂₆ is -(CH₂)ₙ-, wherein n is selected from 0, 1, 2, 3 or 4;
R₃ has the structure of -R₃ₐ-R_{3b}-R_{3c}-R_{3d}-R_{3c}, R₄ has the structure of -R₄ₐ-R_{4b}-R_{4c}-R_{4d}-R_{4c}; R₅ has the structure of -R₅ₐ-R_{5b}-R_{5c}-R_{5d}-R₅ₑ;
wherein, R₃ₐ, R_{3c}, R₄ₐ, R_{4c}, R₅ₐ, R_{5c} are each independently -(CH₂)ₙ-, wherein n is selected from a positive integer ranging from 1 to 14;
R_{3b}, R_{3d}, R_{4b}, R_{4d}, R_{5b}, R_{5d} are each independently absent or selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, -(C=C)-(CH₂)-(C=C)-, -(C=C)-, -CH₂-, -(C≡C)-;
R_{3c}, R_{4c}, R_{5c} are each independently C₂-C₂₀ hydrocarbyl group;
R₆ is H;
R₇ is selected from C₁-C₅ hydrocarbyl group, or -(CH₂)ₘO(CH₂)ₙ-, wherein m, n are each independently selected from 1, 2, 3;
method III comprises the following steps:
(M1) under the protection of an inert gas, compounds C1 and C2 are reacted to obtain C3;
(M2) under the protection of an inert gas, compound C3 undergoes de-Boc deprotection to obtain compound C4;
(M3) under the protection of an inert gas, compound C4 is reacted with C5 or C6 to obtain compound C7, i.e., the compound represented by Formula (I-3); 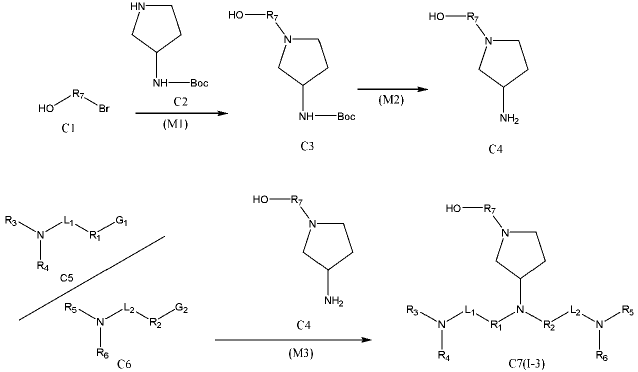
wherein, R₁ and R₂ are each independently selected from -(CH₂)ₙ-, wherein n is a positive integer ranging from 1 to 14;
G₁ and G₂ are each independently selected from an active functional group;
L₁ has the structure of -(L₁ₐ-L_{1b})- from right to left, or is absent, wherein, L₁ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-; L_{1b} is -(CH₂)ₙ-, wherein n is selected from 0, 1, 2, 3 or 4;
L₂ has the structure of -(L₂ₐ-L_{2b})- from left to right, or is absent; wherein, L₂ₐ is selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-; L_{2b} is -(CH₂)ₙ-, wherein n is selected from 0, 1, 2, 3 or 4;
R₃ has the structure of -R₃ₐ-R_{3b}-R_{3c}-R_{3d}-R_{3c}, R₄ has the structure of -R₄ₐ-R_{4b}-R_{4c}-R_{4d}-R_{4c}; R₅ has the structure of -R₅ₐ-R_{5b}-R_{5c}-R_{5d}-R₅ₑ, R₆ has the structure of -R₆ₐ-R_{6b}-R_{6c}-R_{6d}-R₆ₑ;
wherein, R₃ₐ, R_{3c}, R₄ₐ, R_{4c}, R₅ₐ, R_{5c}, R₆ₐ and R_{6c} are each independently -(CH₂)ₙ-, wherein n is selected from a positive integer ranging from 1 to 14;
R_{3b}, R_{3d}, R_{4b}, R_{4d}, R_{5b}, R₅ₐ, R_{6b} and R_{6d} are each independently absent or selected from the following functional groups: -O-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, -(C=C)-(CH₂)-(C=C)-, -(C=C)-, -CH₂-, -(C≡C)-;
R_{3c}, R_{4c}, R_{5c}, R_{6c} are each independently C₂-C₂₀ hydrocarbyl group;
R₇ is selected from C₁-C₅ hydrocarbyl group, or -(CH₂)ₘO(CH₂)ₙ-, wherein m, n are each independently selected from 1, 2, 3.

9. A lipid nanoparticle (LNP), wherein, the lipid nanoparticle comprises the ionizable lipid, or pharmaceutically acceptable salt, tautomer or stereoisomer thereof according to claim 1.

10. A lipid nanoparticle pharmaceutical formulation, wherein, the lipid nanoparticle pharmaceutical formulation comprises:
i) the lipid nanoparticle according to claim 9;
ii) a biologically active substance encapsulated in the lipid nanoparticle; and
iii) a pharmaceutically acceptable carrier.

11. A method for preparing the lipid nanoparticle pharmaceutical formulation according to claim 10, wherein, the method comprises:
(a) mixing the ionizable lipid, or pharmaceutically acceptable salt, tautomer or stereoisomer thereof according to claim 1, and optional auxiliary lipids with an organic solvent to obtain a lipid organic phase;
(b) mixing a biologically active substance with an aqueous solvent to obtain an aqueous phase containing the biologically active substance;
(c) mixing the lipid organic phase in step (a) with the aqueous phase in step (b) to obtain the lipid nanoparticle pharmaceutical formulation.

12. Use of the ionizable lipid, or pharmaceutically acceptable salt, tautomer or stereoisomer thereof according to claim 1 for the manufacture of a drug delivery system.

13. Use of the lipid nanoparticle according to claim 9 in the manufacture of a medicament for treating and/or preventing a tumor, an infectious disease and a rare disease.

14. Use of the ionizable lipid, or pharmaceutically acceptable salt thereof according to claim 1, wherein, it is used for the manufacture of a lipid nanoparticle pharmaceutical formulation, and the lipid nanoparticle pharmaceutical formulation is used for delivering a biologically active substance to cells in a subject in need thereof.
